(19) Europäisches Patentamt / European Patent Office / Office européen des brevets

(11) **EP 3 995 588 B1**

(12) **EUROPEAN PATENT SPECIFICATION**

(45) Date of publication and mention
of the grant of the patent:
**17.12.2025 Bulletin 2025/51**

(21) Application number: **20845892.7**

(22) Date of filing: **28.07.2020**

(51) International Patent Classification (IPC):
***C12Q 1/6806*** (2018.01)    ***C12Q 1/6883*** (2018.01)
***C12Q 1/68*** (2018.01)

(52) Cooperative Patent Classification (CPC):
(C-Sets available)
**C12Q 1/6883; C12N 15/1093; C12Q 1/6806;**
C12Q 2600/156                                    (Cont.)

(86) International application number:
**PCT/CN2020/105117**

(87) International publication number:
**WO 2021/018127 (04.02.2021 Gazette 2021/05)**

(54) **LIBRARY CREATION METHOD AND APPLICATION**

BIBLIOTHEKSERSTELLUNGSVERFAHREN UND -ANWENDUNG

PROCÉDÉ ET APPLICATION DE CRÉATION DE BANQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.07.2019 CN 201910694844**

(43) Date of publication of application:
**11.05.2022 Bulletin 2022/19**

(73) Proprietor: **Genetron Health (Beijing) Co, Ltd.
Beijing 102206 (CN)**

(72) Inventors:
• **ZHENG, Qiaosong**
  **Beijing 102206 (CN)**
• **SHI, Xiao**
  **Beijing 102206 (CN)**
• **JIAO, Yuchen**
  **Beijing 102206 (CN)**
• **CHEN, Min**
  **Beijing 102206 (CN)**
• **ZHANG, Kaihua**
  **Beijing 102206 (CN)**
• **WANG, Sizhen**
  **Beijing 102206 (CN)**
• **YAN, Hai**
  **Beijing 102206 (CN)**

(74) Representative: **Isarpatent
Patent- und Rechtsanwälte Barth
Charles Hassa Peckmann & Partner mbB
Friedrichstrasse 31
80801 München (DE)**

(56) References cited:
WO-A1-2016/074338    WO-A1-2019/076018
CN-A- 104 372 093    CN-A- 105 524 983
CN-A- 106 555 226

• **FIELDS BRYDEN ET AL: "MAUI-seq:
Metabarcoding using amplicons with unique
molecular identifiers to improve error
correction", BIORXIV, 12 January 2020
(2020-01-12), pages 1 - 34, XP093293464,
Retrieved from the Internet <URL:https://www.
biorxiv.org/content/10.1101/538587v3.full.pdf>
DOI: 10.1101/538587**
• **BALECH, B. ET AL.: "Tackling critical parameters
in metazoan meta-barcoding experiments: a
preliminary study based on coxI DNA barcode",
PEERJ, vol. 6, 13 June 2018 (2018-06-13), pages
e4845, XP055777555**
• **BHAGWAT, R.M. ET AL.: "Two New Potential
Barcodes to Discriminate Dalbergia Species",
PLOS ONE, vol. 10, no. 11,, 16 November 2015
(2015-11-16), pages e0142965, XP055777557**

• **ZHAO, HUANYING ET AL.: "Application of Polymerase Chain Reaction-high-resolution Melt Technology for Bacterial Identification in Samples Collected from Lower Respiratory Tract", JOURNAL OF MICROBES AND INFECTIONS, vol. 10, no. 5, 25 October 2015 (2015-10-25), pages 308 - 314, XP055777562**

Remarks:
The complete document including Reference Table(s) and the Sequence Listing(s) can be downloaded from the EPO website

(52) Cooperative Patent Classification (CPC): (Cont.)

C-Sets
**C12N 15/1093, C12Q 2537/143, C12Q 2563/179; C12Q 1/6806, C12Q 2525/191, C12Q 2531/113, C12Q 2535/122, C12Q 2563/179**

**Description**

## TECHNICAL FIELD

The present invention relates to the technical field of molecular biology, and particularly to a library preparation method and application.

## BACKGROUND

Generally, the current sequencing and analysis of a sequence in a target region first requires a library preparation, and methods of library preparation are nothing more than capture library preparation and amplification library preparation.

The capture library preparation is an enrichment library preparation targeted at a relatively large region of a genome, such as a tens or hundreds of gene whole exon regions, while the multiplex amplification library preparation is to perform a target capture and sequencing and analysis on specific hotspot regions, or the whole exon regions of individual genes.

The method for amplification library preparation is to design corresponding specific primers according to a target region. These primers are then used to conduct a multiplex amplification on target sequences. It should be noted that these specific primers will directly carry sequencing adapters or bridging sequences, and then sequencing adapters are added thereto by a secondary PCR amplification, which is the process of a normal amplification library preparation. There are some problems in the application of the existing amplification library preparation methods. For example, the library preparation process is relatively cumbersome, requiring at least two cycles of PCR amplification and two corresponding library purifications, calling for numerous manual operation time that impose high requirements on operators, thus not conducive to popularization. Moreover, primer design and system optimization are relatively complicated; the cost of library preparation is high; and the entire library preparation process is time-consuming. Fields Bryden ET AL (2020-11-29) present a method for metabarcoding using amplicons with unique molecular identifiers to improve error correction.

## SUMMARY

Aiming at various problems existing in the amplification library preparation, the present invention provides the following technical solutions.

One purpose of the present invention is to provide a primer combination for preparing an amplicon library for detecting the variation of a target gene.

The primer combination consisting of:

a forward outer primer F1, a forward inner primer F2, and a reverse primer R that are designed according to a target amplicon;

The forward outer primer F1 is sequentially composed of a sequencing adapter sequence 1, a barcode sequence for distinguishing different samples, and a universal sequence;

The forward inner primer F2 is sequentially composed of a universal sequence and a forward specific primer sequence of the target amplicon (a molecular tag is not required when detecting a tissue sample);

The reverse outer primer R is sequentially composed of a sequencing adapter 2 and a reverse specific primer sequence of the target amplicon.

In the above primer combination, optionally, a molecular tag is required when detecting low frequency mutations, and the forward inner primer F2 is sequentially composed of a universal sequence, a molecular tag sequence, and a forward specific primer sequence of the target amplicon.

The molecular tag sequence is composed of 6-30 bases, consisting random bases and 0-N (N is an integer $\geq 0$) set(s) of specific bases; the specific bases are set in the random bases, for example, 1 set, 2 sets, 3 sets, or 4 sets; the specific bases in each set are composed of 1-5 bases, such as 1 base, 2 bases, 3 bases, 4 bases, or 5 bases.

The base sequence of each set is randomly selected, and the molecular tag sequence is used to distinguish different starting DNA template molecules. In a library preparation process, except for the fixed position and constant composition of the specific bases in the molecular tag sequence, the types of bases (A, T, G, C) of the random bases can be selected at will.

For example, in an embodiment of the present invention, the specific bases are set as 1 or 2 sets, with the sequence of ACT and/or TGA; for example, in the present embodiment, the molecular tag sequence is NNNNNACTNNNNTGA, where ACT and TGA are the specific bases, N is a random base of A, T, C, or G.

In the above primer combination, the sequencing adapter 1 and the sequencing adapter 2 are corresponding sequencing adapters selected according to different sequencing platforms.

In the above primer combination,

the sequencing platform is an Illumina platform, the sequencing adapter 1 is I5, and the sequencing adapter 2 is I7;

or the sequencing platform is an Ion Torrent platform, the sequencing adapter 1 is A, and the sequencing adapter 2 is P;

or the sequencing platform is a BGI/MGI platform;

or, the nucleotide sequence of the universal sequence is shown in SEQ ID NO: 1.

Another purpose of the present invention is to provide a kit for preparing an amplicon library for detecting the variation of a target gene, wherein the kit provided by the present invention includes the above-mentioned primer combination.

The above kit further includes a polymerase chain reaction (PCR) amplification buffer and a DNA polymerase system.

Another purpose of the present invention is the use of the primer combination or the kit described above:

(1) an application in preparing the amplicon library for detecting the variation of the target gene;

(2) an application in detecting mutation sites or variations in a target region of a sample to be tested;

(3) an application in detecting a variation frequency of the target region of the sample to be tested.

Another purpose of the present invention is to provide a method of preparing an amplicon library for detecting a variation of a target gene, wherein the method includes the following steps:

taking the DNA or cDNA of a sample to be tested as a template, carrying out a one-step PCR amplification using the above primer combination or the above kit to obtain an amplified product, i.e., the amplicon library of the target gene.

In the above method, the molar ratio of the forward outer primer F1, the forward inner primer F2, and the reverse primer R in an amplification system for the one-step PCR amplification is (5-20):(1-20):(5-20).

In the above method, the sample to be tested is a tissue sample, a frozen sample, a puncture sample, a formalin-fixed paraffin-embedded (FFPE) sample, blood, urine, cerebrospinal fluid, pleural fluid, or other body fluids.

The application of the above method in detecting mutation sites or variations of the target gene of the sample to be tested.

The application of the above method in detecting a variation frequency of the target gene of the sample to be tested.

The amplicon library prepared by the above method also falls within the protection scope of the present invention.

Another purpose of the present invention is to provide a method for detecting the variation of the target gene of the sample to be tested, wherein the method includes the following steps:

1) preparing an amplicon library of the target gene by the above method;

2) evenly mixing the amplicon libraries of the target genes of all samples, and then diluting to obtain a sequencing DNA library;

3) sequencing the sequencing DNA library to obtain a sequencing result, and analyzing the variation of the target gene of the sample to be tested according to the sequencing result.

Another purpose of the present invention is to provide a method of detecting a variation frequency in a target region of a sample to be tested, wherein the method includes the following steps:

1) preparing an amplicon library of the target gene by the above method;

2) evenly mixing the amplicon libraries of the target genes of all samples, and then diluting to obtain a sequencing DNA library;

3) sequencing the sequencing DNA library to obtain a sequencing result, and calculating the variation frequency of the target gene of the sample to be tested according to the sequencing result.

Variation frequency=number of mutation clusters/total number of effective clusters×100%.

In the above method, the sample to be tested is an in vitro tissue sample, a frozen sample, a puncture sample, an FFPE sample, blood, urine, cerebrospinal fluid, or pleural fluid.

In the above method, optionally,

the nucleotide sequence of the universal sequence is shown in SEQ ID NO: 1;

the nucleotide sequence of the sequencing adapter 1 is shown in SEQ ID NO: 2;

the nucleotide sequence of the sequencing adapter 2 is shown in SEQ ID NO: 17.

For example, when the target gene to be tested is EGFR, optionally, the corresponding forward specific primer sequence and reverse specific primer sequence are respectively shown in SEQ ID NO: 14 and SEQ ID NO: 18, or, SEQ ID NO: 15 and SEQ ID NO: 19, or, SEQ ID NO: 21 and SEQ ID NO: 24, or, SEQ ID NO: 22 and SEQ ID NO: 25;

When the target gene to be tested is ERBB2, optionally, the corresponding forward specific primer sequence and reverse specific primer sequence are respectively shown in SEQ ID NO: 16 and SEQ ID NO: 20, or, SEQ ID NO: 23 and SEQ ID NO: 26;

When the target gene to be tested is EML4, optionally, the corresponding forward specific primer sequence and reverse specific primer sequence are respectively shown in SEQ ID NO: 27 and SEQ ID NO: 31, or, SEQ ID NO: 28 and SEQ ID NO: 31;

When the target gene to be tested is LMNA, optionally, the corresponding forward specific primer sequence and reverse specific primer sequence are respectively shown in SEQ ID NO: 29 and SEQ ID NO: 32;

When the target gene to be tested is MYC, optionally, the corresponding forward specific primer sequence and reverse specific primer sequence are respectively shown in SEQ ID NO: 30 and SEQ ID NO: 33.

For example, the barcode sequences are all nucleotides with a length of 6-12 nt, no more than 3 consecutive bases, and a GC content of 40-60%;

The universal sequence 1 and the universal sequence 2 generally have a length of 16-25 nt, and a GC content of 35-65%, without consecutive bases or obvious secondary structure;

For example, the molecular tag sequence is a sequence containing 6-15 random bases; including but not limited to the above sequences; in the embodiment of the present invention, for example, the barcode sequences for distinguishing different samples are shown in SEQ ID NO: 3 to SEQ ID NO: 12;

The variation can be point mutation, deletion or insertion, or fragment fusion.

## BRIEF DESCRIPTION OF THE DRAWINGS

FIG. 1 shows the composition of primers used for a one-step rapid amplification library preparation technology.
FIG. 2 shows products obtained when amplifying a template by the rapid amplification library preparation technology.
FIG. 3 shows an Agilent 2200 result of the library prepared by a BRCA1/2 one-step primer pool.
FIG. 4 shows the homogeneity of sequencing amplicons of the library prepared by the BRCA1/2 one-step primer pool.
FIG. 5 is a schematic diagram showing the functional structure of each component of a quadruple-functional primer and a triple-functional primer.
FIG. 6 shows homogeneity results of the libraries prepared by a triple-functional component primer pool and a quadruple-functional component primer pool.
FIG. 7 shows the number of clusters (the number of molecular tag types) of one of the amplicons obtained after data analysis of the library prepared by using 30 ng cfDNA and one-step primer pool.
FIG. 8 shows the background noises at the level of 0.1‰-1‰ after sequencing the libraries prepared by the two methods.
FIG. 9 shows a result of Agilent 2200 TapeStation of the library prepared in Embodiment 2.
FIG. 10 shows a result of Agilent 2200 TapeStation of the library prepared in Embodiment 3.
FIG. 11 shows a result of Agilent 2200 TapeStation of the library prepared in Embodiment 4.

## DETAILED DESCRIPTION OF THE EMBODIMENTS

The experimental methods used in the following embodiments, unless otherwise specified, are all conventional methods.

The materials, reagents, etc. used in the following embodiments, unless otherwise specified, are commercially available.

Embodiment 1. Design and synthesis of primers for one-step amplicon sequencing library preparation

I. Design of primers for one-step amplicon sequencing library preparation

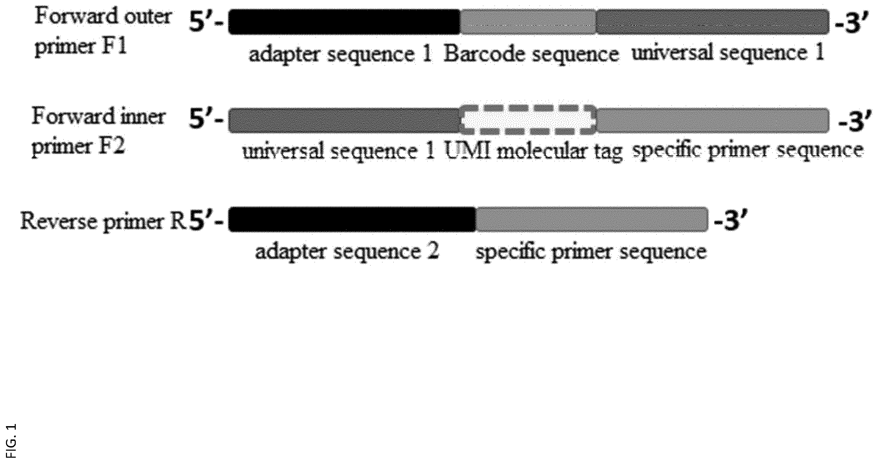

The present invention provides an amplification library preparation method to prepare a second-generation sequencing library, and the structures of primers involved in the method are as follows (see FIG. 1):

forward outer primer F1: 5'-sequencing adapter sequence 1 + Barcode sequence + universal sequence -3';
forward inner primer F2: 5'-universal sequence + molecular tag sequence + gene forward specific primer sequence-3';
or forward inner primer F2: 5'-universal sequence + gene forward specific primer sequence-3' (molecular tag is required when detecting low frequency mutations, and molecular tag is not required when detecting tissue samples);
reverse primer R: 5'-sequencing adapter sequence 2 + gene reverse specific primer sequence -3'.

When detecting low-frequency mutations, the structure of the forward inner primer F2 is: 5'-universal sequence + molecular tag sequence + gene forward specific primer sequence-3'.

Among them, the barcode sequence is a nucleic acid sequence that is used to distinguish different samples; a sample to be tested corresponds to a barcode sequence. The barcode sequence is 6-12 nt in length, and has no more than 3 consecutive bases, and a GC content of 40-60%, and the primer where the Barcode sequence is introduced has no obvious secondary structure, etc.

The forward outer primer F1 is used to distinguish different samples. The same sample has the same forward outer primer F1 regardless of detection sites.

The molecular tag sequence is used to mark different starting DNA template molecules (templates of different amplicons), and a starting DNA template molecule corresponds to a molecular tag sequence.

The molecular tag sequence includes random bases and at least one set of specific bases, the specific bases are set in the random bases, for example, 1 set or 2 sets; each set of specific bases is composed of 1-5 bases, for example, 3 bases or 4 bases. In a library preparation process, except for the fixed position and constant composition of the specific bases in the molecular tag sequence, the types of bases (A, T, G, C) of the random bases are randomly selected.

The starting templates of the sequencing results are classified using the molecular tag sequences, which can eliminate amplification errors and sequencing errors. In the present embodiment, two types of specific bases are used: ACT and TGA, which can be used separately or in combination.

Gene forward specific primer sequence and gene reverse specific primer sequence are primer sequences (respectively including the required forward primers and corresponding reverse primers to amplify different target regions) used to amplify specific target regions;

The universal sequence 1 is a specific nucleic acid sequence, which can be changed according to actual needs. The universal sequence 1 has a length of 16-25 nt, and a GC content of 35-65%, without consecutive bases or obvious secondary structure.

In present embodiment, the universal sequence used is GGCACCCGAGAATTCCA (SEQ ID NO: 1), with a length of 17 nt;

The sequencing adapter sequence 1 and the sequencing adapter sequence 2 are specific sequences that need to be introduced to primers during sequencing, and can specifically correspond to Ion Torrent, Illumina, or BGISEQ/MGISEQ sequencing platforms.

If the sequencing platform is the Illumina platform, the sequencing adapter sequences 1 and 2 are I5 and I7, respectively, and the adapter sequences are complementary to the primer sequences on the chip. The adapter is introduced to link a nucleic acid fragment to a vector.

If the sequencing platform is the Ion Torrent platform, the sequencing adapter sequences 1 and 2 are A and P, respectively, the A adapter is used for sequencing and complementary to the sequencing primer, and the P adapter is complementary to the sequence on the vector, so as to link a template to the vector.

If the sequencing platform is the BIISEQ/MGISEQ platform, the sequencing adapters are required for sequencing, which are specific sequences meeting the requirements of single-strand circularization, subsequent DNB preparation, and sequencing.

When the second-generation sequencing library is used in a sequencing, multiple samples will be tested simultaneously. As such, a set of forward outer primers F1 will be designed. M forward outer primers F1 correspond to M samples, and the barcode sequence in each forward outer primer F1 is different; P forward inner primers F2 and corresponding Q (generally P=Q, but there are also situations where P does not equal Q, for example, in a detection of RNA fusion genes) reverse primers R are designed according to the number P of amplicons required for the target capture region on each sample, and the structures of the molecular tags in the P forward inner primers F2 are identical.

II. Amplification principle of one-step amplicon sequencing library

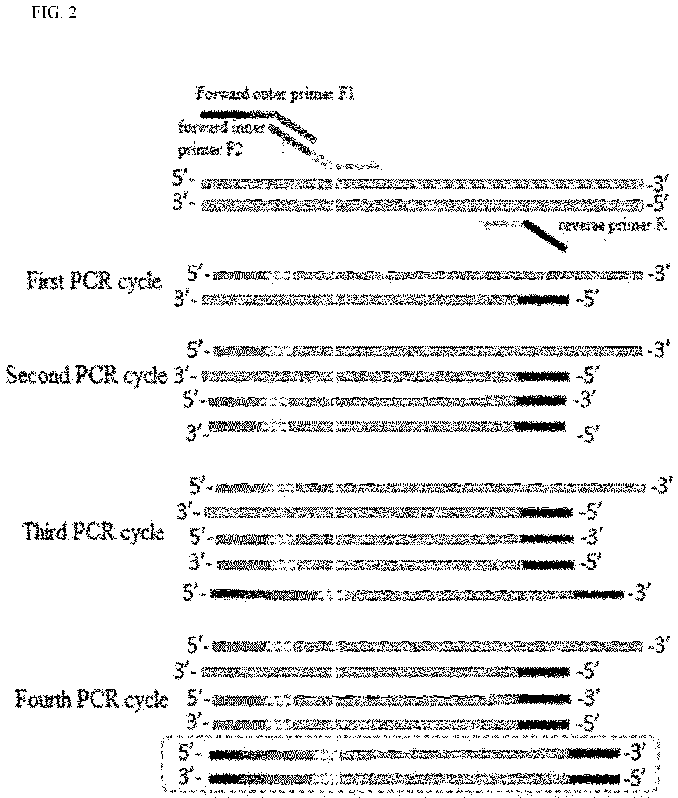

The primers design of one-step rapid amplification library preparation technology are as described above. When amplifying a template DNA/RNA, the procedure shown in FIG. 2 is followed. The forward outer primer F1 and the forward inner primer F2 share a normal universal sequence, so the forward outer primer F1 can use the forward inner primer F2 as a template to add a sequencing adapter and a sample barcode sequence to a target sequence. During amplification, the forward inner primer MIX1 (MIX1 is formed by mixing the forward inner primers F2 of multiple amplicons at a specific ratio) and the reverse primer MIX2 (MIX2 is formed by mixing the reverse primers R corresponding to multiple amplicons at a specific ratio) are used to perform the first cycle of reaction on the template to produce amplified products with F2 and R; in the second cycle of reaction, in addition to the above two PCR products, products with F2 and R sequences respectively at both ends will be further obtained; in the third cycle of reaction, a target product with the complete sequence of the complete sequencing library begins to appear, but at this time, the product has only one strand; subsequently in the fourth cycle of reaction, a double-stranded product with complete adapter sequences at two ends will be produced. Since the forward outer primer F1 has a much higher TM value and a much higher concentration than the forward inner primer F2, exponential amplifications of the complete products (that is, the two products marked with the red dashed box in the products of the fourth PCR cycle) will be realized later. Finally, the library preparation is completed after a dozen to dozens of cycles of reaction processes.

III. Establishment of detection method

1. One-step amplification

The primers synthesized in section I above were prepared as follows:
The forward outer primer F1 was dissolved in water to a primer concentration of 100 $\mu$M, and the forward inner primers F2 were respectively dissolved in water to a primer concentration of 100 $\mu$M. Subsequently, the various primers were mixed at an equimolar ratio to form the forward outer primer MIX1. The reverse primers R were respectively dissolved in water to 100 $\mu$M, and then mixed at an equimolar ratio into the reverse primer MIX2.

The genomic DNA of multiple samples to be tested was extracted.

The reagents shown in Table 1 were successively added to a 0.2 ml eight-row tube or 96-well plate (each type of nucleic acid sample was extracted according to the instruction of the specific manufacturer's kit provided in the embodiment):

Table 1 shows the amplification system of a certain sample

| Reagent | Volume ($\mu$l) |
| --- | --- |
| KAPA HiFi PCR Kits (including but not limited to the DNA polymerase) | 10 |
| Genomic DNA of a certain sample (generally 5-20 ng of gDNA) | 1-10 |
| Forward inner primer MIX1 (100 $\mu$M) | 0.01-5 |
| Forward outer primer F1 (100 $\mu$M) | 0.01-5 |
| Reverse primer MIX2 (100 $\mu$M) | 0.01-5 |
| DNAase-free H$_2$O | Replenish water to 20 |

The procedure of the above PCR amplification is shown in Table 2.

Table 2 shows the PCR amplification procedure

| Temperature | Time | Number of cycles |
| --- | --- | --- |
| 95°C | 2 m | |
| 95°C | 30 s | |
| 60°C | 90 s | 15-30 |
| 72°C | 90 s | |
| 72°C | 10m | |
| 4°C | --- | |

After the PCR reaction was completed, the PCR product obtained was the amplicon library.

2. Magnetic bead purification and Qubit quantification

After the PCR reaction was completed, the Agencourt AMPure XP Kit (Cat. No. A63880/A63881/A63882) from Beckman Coulter Inc. was used for purification. The operation steps were as follows:

1) The Agilent court AMPure XP Kit was taken out 30 min in advance, fully vortex and put aside at room temperature.

2) After the PCR reaction, the magnetic beads were fully vortexed again, 24 μl of magnetic beads were added to the system, blow repeatedly more than 5 times or vortex fully , and put aside at room temperature for 5 min.

3) The Eppendorf (EP) tubes were transferred to a magnetic stand and put aside for 5 min until the solution was clear, by using a pipette to carefully remove the supernatant without contacting the magnetic beads.

4) 100 μl of freshly prepared 80% ethanol solution was added to each tube, the EP tubes were slowly rotated for 2 cycles on the magnetic stand, followed by putting aside for 5 min and discarding the supernatant.

5) The step 4 was repeated one more time.

6) The EP tubes were opened and put aside at room temperature to allow a complete liquid volatilization until surfaces of the magnetic beads became matte. The magnetic beads should not be dried excessively.

7) The EP tubes were removed from the magnetic stand, 30 μl of PCR-grade purified water was added, followed by vortex to mix well, and putting aside at room temperature for 10 min.

8) The EP tubes in the previous step were placed on the magnetic stand for 2 min or until the solution was clear, followed by using a pipette to carefully absorb the supernatant on the side away from the magnet without contacting the magnetic beads.

A purified amplicon library was obtained.

The purified amplicon library was subjected to a DNA library concentration determination and an Agilent 2200 TapeStation Systems detection using Qubit 2.0.

3. Sequencing and result analysis

The purified amplicon libraries of multiple samples were mixed at an equal concentration, and then diluted to 100 PM to obtain a DNA library for amplicon sequencing. Sequencing was performed (sequenator used was Ion GeneStudio™ S5 Plus System, Thermofisher, A38195), after data processing and analysis (S5 Torrent Server), the mutations and mutation frequency of a tested sample were obtained.

The calculation method of the variation frequency of the library with molecular tags was as follows:
Since the original template was subjected to molecular marking during the library amplification process, the calculation method of the mutation frequency was as follows:

In the sequencing results, DNA molecules with the same kind of molecular tags were defined as a cluster, and DNA molecules with the same kind of molecular tags were amplified products of an initial DNA template, that is, a series of DNA molecules obtained by amplification using the same original template;

Whether mutations occurred in the cluster or not was confirmed. If the proportion of a specific type of bases in a certain position in the cluster was greater than or equal to 80%, the cluster was recorded as an effective cluster. If the number of mutant DNA molecules with molecular tags in the effective cluster accounted for greater than or equal to 80%, it was recorded as a mutation cluster;

Variation frequency=number of mutation clusters/total number of effective clusters×100%.

Notes: It is statistically significant only when the number of DNA molecules in the same cluster (a sequence sequenced) in the sequencing results is ≥2.

Embodiment 2. Preparation and sequencing of one-step amplicon sequencing library

1. Design of primers for one-step amplicon sequencing library preparation

The detection region of this experiment contained three amplicons (EGFR L858R, 19del and insertion mutations of ERBB2);
The test samples included two frozen lung cancer tissue samples (sample 1, sample 2), four lung cancer FFPE (formalin fixed paraffin-embedded tissue samples) samples (sample 3, sample 4, sample 5, sample 6), and two white blood cell samples from healthy subjects (sample 7, sample 8). The mutations of the above eight samples were already known.

The primers (eight Barcode sequences were used in the present embodiment) shown in Table 3 were designed according to the three amplicons (EGFR L858R, 19del and insertion mutations of ERBB2):

Table 3 shows the primer sequences of EGFR L858R, 19del and insertion mutations of ERBB2

| Required primer | | Amplicon | Primer sequence |
|---|---|---|---|
| Forward outer primer F1 | Sequencing adapter sequence 1 | | CCATCTCATCCCTGCGTGTCTCCGACTCAG (SEQ ID NO: 2) |
| | Barcode sequence (ten listed) | | TCCTCGAATC (SEQ ID NO: 3) |
| | | | TAGGTGGTTC (SEQ ID NO: 4) |
| | | | TCTAACGGAC (SEQ ID NO: 5) |
| | | | TTGGAGTGTC (SEQ ID NO: 6) |
| | | | TCTAGAGGTC (SEQ ID NO: 7) |
| | | | TCTGGATGAC (SEQ ID NO: 8) |
| | | | TCTATTCGTC (SEQ ID NO: 9) |
| | | | AGGCAATTGC (SEQ ID NO: 10) |
| | | | TTAGTCGGAC (SEQ ID NO: 11) |
| | | | CAGATCCATC (SEQ ID NO: 12) |
| | Universal sequence | | GGCACCCGAGAATTCCA (SEQ ID NO: 1) |
| Forward inner sequence F2 | Universal sequence | | GGCACCCGAGAATTCCA (SEQ ID NO: 1) |
| | Gene forward specific primer sequence | EGFR L858R | CAGGAACGTACTGGTGAAAACAC (SEQ ID NO: 14) |
| | | EGFR 19del | CTTCCTTCTCTCTCTGTCATAGGGA (SEQ ID NO: 15) |
| | | ERBB2 | CTCCCATACCCTCTCAGCGTA (SEQ ID NO: 16) |
| Reverse primer R | Sequencing adapter sequence 2 | | CCTCTCTATGGGCAGTCGGTGAT (SEQ ID NO: 17) |
| | Gene reverse specific primer sequence | EGFR L858R | GAAAATGCTGGCTGACCTAAAGC (SEQ ID NO: 18) |
| | | EGFR 19del | AGCAAAGCAGAAACTCACATCGA (SEQ ID NO: 19) |
| | | ERBB2 | AGCCATAGGGCATAAGCTGTG (SEQ ID NO: 20) |

The sequencing adapter is suitable for the Ion GeneStudio™ S5 Plus System sequencing platform.

II. One-step amplicon sequencing library

Nucleic acid extraction and purification kit (DNA extraction from FFPE samples: GeneRead DNA FFPE kit, Qiagen, 180134; DNA extraction from frozen tissue samples: QIAamp DNA Mini Kit 250, QIAGEN, 51306).

1. One-step amplification

The PCR product was obtained according to step 1 in section III of Embodiment 1.
The amplification system is shown in Table 4.

Table 4 shows the amplification system

| Reagent | Volume (µl) |
|---|---|
| KAPA HiFi PCR Kits (including but not limited to the DNA polymerase) | 10 |
| Genomic DNA of a certain sample | 5-20 |

(continued)

| Reagent | Volume ($\mu$l) |
|---|---|
| Forward inner primer MIX1 (100 $\mu$M) | 1 |
| Forward outer primer F1 (100 $\mu$M) | 0.5 |
| Reverse primer MIX2 (100 $\mu$M) | 0.5 |
| DNAase-free $H_2O$ | Replenish water to 20 |

Table 5 shows the amplification procedure

| Temperature | Time | Number of cycles |
|---|---|---|
| 95°C | 2 m | |
| 95°C | 30 s | |
| 60°C | 90 s | 18 |
| 72°C | 90 s | |
| 72°C | 10m | |
| 4°C | --- | |

2. Magnetic bead purification and Qubit quantification

Same steps were performed as those in step 2 of section III of Embodiment 1.

The PCR product was purified and recovered by the magnetic bead (Agencourt AMPure XP, Beckman Coulter, A63880), and DNA library concentration determination and Agilent 2200 TapeStation Systems detection were conducted using Qubit 2.0.

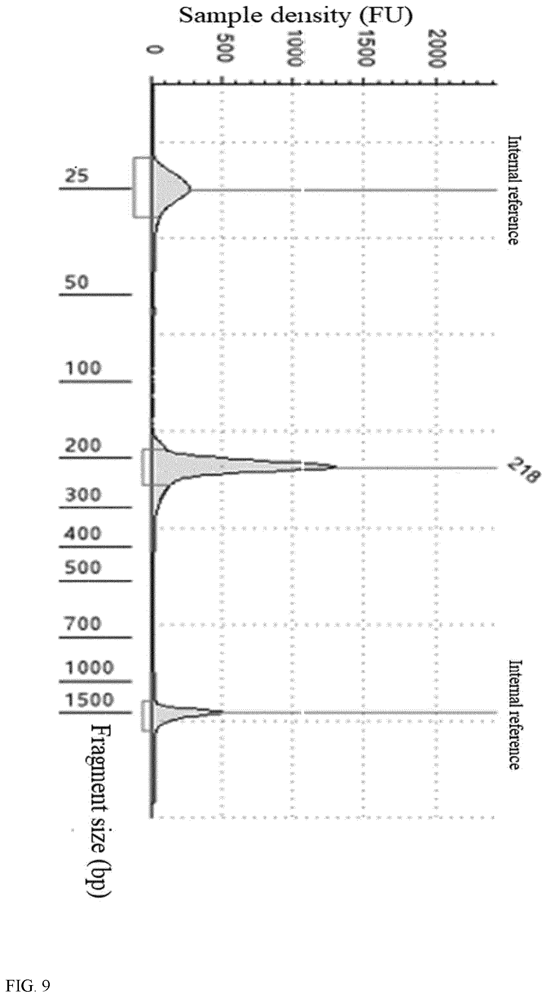

The result of the Agilent 2200 TapeStation Systems detection is shown in FIG. 9.

3. Sequencing and result analysis

The PCR products of all samples were mixed at an equal concentration and diluted to 100 pM to obtain a DNA library for sequencing.

The sequencing results are shown in Table 6:

Table 6 shows the results of sequencing

| Sample No. | Method of the present invention | | 63 gene detection | |
|---|---|---|---|---|
| | Variation type | Variation frequency | Variation type | Variation frequency |
| Frozen sample 1 | EGFR:L858R | 13.7% | EGFR:L858R | 17.8% |
| Frozen sample 2 | EGFR:p.E746_A750delELREA | 8.1% | EGFR:p.E746_A750delELREA | 7.3% |
| FFPE sample 1 | EGFR:L858R | 33.5% | EGFR:L858R | 31.9% |
| FFPE sample 2 | EGFR:L858R | 21.0% | EGFR:L858R | 19.2% |
| FFPE sample 3 | EGFR:p.K745_E749delKELRE | 23.8% | EGFR:p.K745_E749delKELRE | 23.5% |
| FFPE sample 4 | ERBB2:p.A775_G776insYVMA | 17.2% | ERBB2:p.A775_G776insYVMA | 17.1% |
| White blood cell sample 1 from healthy subject | None | 0 | None | 0 |

(continued)

| Sample No. | Method of the present invention | | 63 gene detection | |
|---|---|---|---|---|
| | Variation type | Variation frequency | Variation type | Variation frequency |
| White blood cell sample 2 from healthy subject | None | 0 | None | 0 |

EGFR: p.E746_A750delELREA indicates a deletion of the 746th-750th amino acids ELREA (E: Glu glutamic acid; L: Leu leucine; R: Arg arginine; E: Glu glutamate; A: Ala alanine) of the EGFR gene, which is a kind of EGFR 19del;

EGFR: p.K745_E749delKELRE indicates a deletion of the 745th-749th amino acids KELRE (K: Lys lysine; E: Glu glutamic acid; L: Leu leucine; R: Arg arginine; E: Glu glutamic acid) of the EGFR gene, which is a kind of EGFR 19del;

ERBB2: p.A775_G776insYVMA indicates an insertion of YVMA (Y: Tyr Tyrosine; V: Val Valine; M: Met Methionine; A: Ala alanine) between the 775th alanine (A) and the 776th glycine (G) of the ERBB2 gene, corresponding to ERBB2 in Table 3.

The 63 gene detection product is a product of tumor liquid biopsy of Genetron Health (Beijing) Co., Ltd. It targets all solid tumor patients and applies high-throughput and high-precision second-generation sequencing technology to comprehensively detect mutations of 63 gene loci closely related to tumor-targeted therapy and occurrence and development (including mutation analysis of 58 genes, rearrangement analysis of 10 genes, and CNV detection of 7 genes), covering the target region with a sequencing depth of 20,000 x, and reaching a detection sensitivity of 0.1%, which provides comprehensive and high-value reference information for precise medication, molecular typing, and curative effect and recurrence monitoring.

The above results show that the library prepared by the method of the present invention, when used for sequencing, leads to the variation information of tested tissue samples including point mutations, deletion mutations and insertion mutations consistent with that obtained by the known 63 gene detection.

Embodiment 3. Preparation and sequencing of one-step amplicon sequencing library

The samples in this experiment were plasma samples from lung cancer patients, including plasma samples from four different patients and two healthy subjects (the variations of the samples were already known), cfDNA was extracted using the kit (MagMAX™ Cell-Free DNA Isolation Kit, Applied Biosystems™, A29319), and the library was prepared using a primer pool with molecular tags containing EGFR L858R, 19del and insertion mutations of ERBB2.

I. Design of primers for one-step amplicon sequencing library preparation

The primers (forward outer primers were identical, others were different, and six barcode sequences were used in the present embodiment) shown in Table 7 were designed according to three amplicons (EGR L858R, 19del and insertion mutations of ERBB2):

Table 7 shows the primer sequences of EGR L858R, 19del and insertion mutations of ERBB2

| Require primer | | Amplicon | Primer sequence |
|---|---|---|---|
| Forward inner primer F2 | Universal sequence 1 | | GGCACCCGAGAATTCCA (SEQ ID NO: 1) |
| | Molecular tag sequence | | NNNNNACTNNNNTGA (SEQ ID NO: 13), where the bold letters are specific bases. |
| | Gene forward specific primer sequence | EGFR L858R | GGAGGACCGTCGCTTG (SEQ ID NO: 21) |
| | | EGFR 19del | GTGAGAAAGTTAAAATTCCCGTC (SEQ ID NO: 22) |
| | | ERBB2 | CCCATACCCTCTCAGCGT (SEQ ID NO: 23) |
| Reverse | Sequencing | | CCTCTCTATGGGCAGTCGGTGAT (SEQ |

(continued)

| Require primer | | Amplicon | Primer sequence |
|---|---|---|---|
| primer R | adapter sequence 2 | | ID NO: 17) |
| | Gene reverse specific primer sequence | EGFR L858R | CTTCTGCATGGTATTCTTTCTCTTCC (SEQ ID NO: 24) |
| | | EGFR 19del | CACACAGCAAAGCAGAAAC (SEQ ID NO: 25) |
| | | ERBB2 | CCAGAAGGCGGGAGACATATG (SEQ ID NO: 26) |

II. One-step amplicon sequencing library

Nucleic acid extraction and purification kit (DNA extraction from FFPE samples: GeneRead DNA FFPE kit, Qiagen, 180134; DNA extraction from frozen tissue samples: QIAamp DNA Mini Kit 250, QIAGEN, 51306).

1. One-step amplification

The PCR product was obtained according to step 1 in section III of Embodiment 1.

Table 8 shows the amplification system

| Reagent | Volume ($\mu$l) |
|---|---|
| KAPA HiFi PCR Kits (including but not limited to the DNA polymerase) | 10 |
| Genomic DNA of a certain sample | 5-20 |
| Forward inner primer MIX1 (100 $\mu$M) | 0.5 |
| Forward outer primer F1 (100 $\mu$M) | 1 |
| Reverse primer MIX2 (100 $\mu$M) | 1 |
| DNAase-free $H_2O$ | Replenish water to 20 |

Table 9 shows the amplification procedure

| Temperature | Time | Number of cycles |
|---|---|---|
| 95°C | 2 m | |
| 95°C | 30 s | |
| 65°C | 30 s | |
| 62°C | 30 s | 2 |
| 59°C | 30 s | |
| 72°C | 30 s | |
| 95 °C | 30 s | |
| 60°C | 30 s | 16 |
| 72 °C | 30 s | |
| 72 °C | 10m | |
| 4°C | --- | |

2. Magnetic bead purification and Qubit quantification

Same steps were performed as those in step 2 of section III of Embodiment 1.
The PCR product was purified and recovered by the magnetic bead (Agencourt AMPure XP, Beckman Coulter, A63880), and detected by Qubit 2.0 and Agilent 2200 TapeStation Systems.

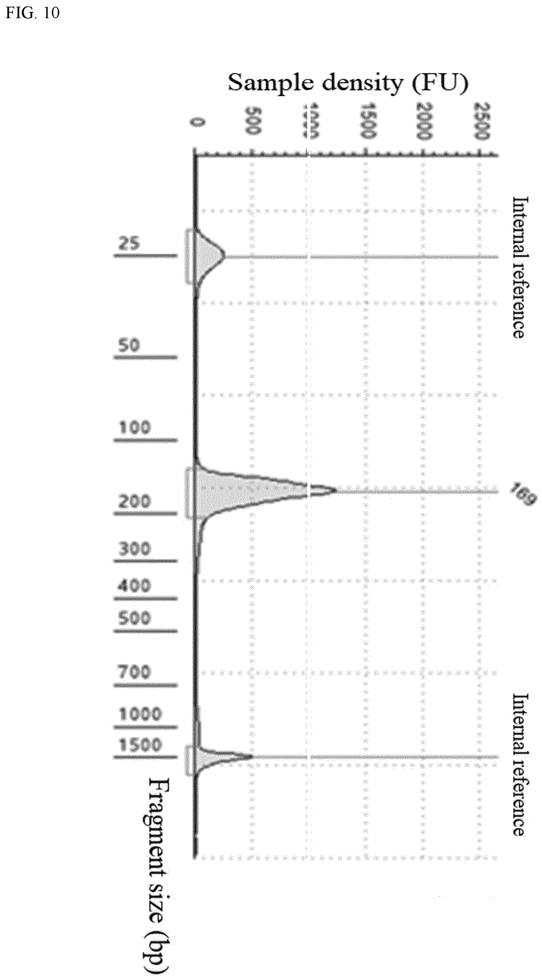

The result of the Agilent 2200 TapeStation Systems is shown in FIG. 10.

3. Sequencing and result analysis

The PCR products of all samples were mixed at an equal concentration and diluted to 100 $\mu$M to obtain a DNA library for amplicon sequencing.

The sequencing results are shown in Table 10:

Table 10 shows the detection results of four tissue samples and two samples from healthy subjects

| Sample No. | Method of the present invention | | 63 gene detection | |
|---|---|---|---|---|
| | Variation type | Variation frequency | Variation type | Variation frequency |
| Patient 1 | EGFR:L858R | 0.57% | EGFR:L858R | 0.72% |
| Patient 2 | EGFR:L858R | 0.21% | EGFR:L858R | 0.18% |
| Patient 3 | EGFR:p.E746_A 750delELREA | 0.80% | EGFR:p.E746_A750 delELREA | 0.93% |
| Patient 4 | ERBB2:p.A775_ G776insYVMA | 0.38% | ERBB2:p.A775_G7 76insYVMA | 0.35% |
| Healthy subject 1 | None | 0 | None | 0 |
| Healthy subject 2 | None | 0 | None | 0 |
| EGFR: p.E746_A750delELREA indicates a deletion of the 746th-750th amino acids ELREA (E: Glu glutamic acid; L: Leu leucine; R: Arg arginine; E: Glu glutamate; A: Ala alanine) of the EGFR gene, which is a kind of EGFR 19del; ERBB2: p.A775_G776insYVMA indicates an insertion of YVMA (Y: Tyr Tyrosine; V: Val Valine; M: Met Methionine; A: Ala alanine) between the 775th alanine (A) and the 776th glycine (G) of the ERBB2 gene, corresponding to ERBB2 in Table 7. | | | | |

The library prepared by the method of the present invention, when used for sequencing, leads to variation information of tested plasma cfDNA samples including point mutations, deletion mutations and insertion mutations consistent with that obtained by the known 63 gene detection. The amount of ctDNA extracted from the patient 1 sample is large. After the patient 1 sample is diluted by 5 times, the detection of L858R with a frequency of 4.6‰ is still obtained (after deduplicating the data Reads: mutation cluster = 2; total cluster at the locus = 4380).

Embodiment 4. Preparation and sequencing of one-step amplicon sequencing library

The samples in this experiment were fine-needle aspiration (FNA) puncture samples of 3 thyroid cancer patients with gene fusion (gene fusion information was already known) and FNA puncture samples of 2 patients with benign thyroid nodules. RNA samples were extracted using MagMAX™ FFPE DNA/RNA Ultra Kit (Applied Biosystems™, A31881) according to the manufacturer's instruction, and then reverse transcription was conducted using SuperScript™ VILO™ MasterMix (Invitrogen™, 11755050) according to the manufacturer's kit instruction.

I. Design of primers for one-step amplicon sequencing library preparation

The primers (forward outer primers were identical to those in Table 3, others were different, and five barcode sequences were used in the present embodiment) shown in Table 11 were designed according to gene fusion: the primers for detecting gene fusion were designed before and after the breakpoint, and there was no fixed forward and reverse primer matching; the forward and reverse primers designed for the fusion breakpoint were shown as below, ALK_20 and ELM4_6/EML4_13 were combined separately to detect two ALK-EML4 fusion forms .

Table 11 shows the primers of gene fusion

| Require primer | | Amplicon | Primer sequence |
|---|---|---|---|
| Forward inner primer F2 | Universal sequence 1 | | GGCACCCGAGAATTCCA (SEQ ID NO: 1) |
| | Gene forward specific primer sequence | EML4_6_ | ACTGCAGACAAGCATAAAGATGTCA (SEQ ID NO: 27) |
| | | EML4_13 | ACTACTGTAGAGCCCACACCTG (SEQ ID NO: 28) |
| | | LMNA | CTGAGAACAGGCTGCAGACC (SEQ ID NO: 29) |
| | | MYC | CCTGGTGCTCCATGAGGAGA (SEQ ID NO: 30) |
| Reverse primer R | Sequencing adapter sequence 2 | | CCTCTCTATGGGCAGTCGGTGAT (SEQ ID NO: 17) |
| | Gene reverse | ALK_20 | CTCAGCTTGTACTCAGGGCTC (SEQ |
| | specific primer sequence | | ID NO: 31) |
| | | LMNA | ACTCACGCTGCTTCCCATT (SEQ ID NO: 32) |
| | | MYC | GTGATCCAGACTCTGACCTTTTGC (SEQ ID NO: 33) |

II. One-step amplicon sequencing library

1. One-step amplification

The PCR product was obtained according to step 1 in section III of Embodiment 1.

Table 12 shows the amplification system

| Reagent | Volume ($\mu$l) |
|---|---|
| Platinum multiplex PCR Master Mix | 15 |
| Thy RNA Fusion Panel | 2 |
| Barcode (50 $\mu$M) | 1 |
| cDNA | $\leq$12 |
| ddH$_2$O | Replenish to 30 |

Table 13 shows the amplification procedure

| Temperature | Time | Number of cycles |
|---|---|---|
| 95°C | 2 min | |
| 95°C | 30 s | |
| 60°C | 90 s | 18 |
| 72°C | 90 s | |
| 72°C | 10 min | |
| 4°C | $\infty$ | |

2. Magnetic bead purification and Qubit quantification

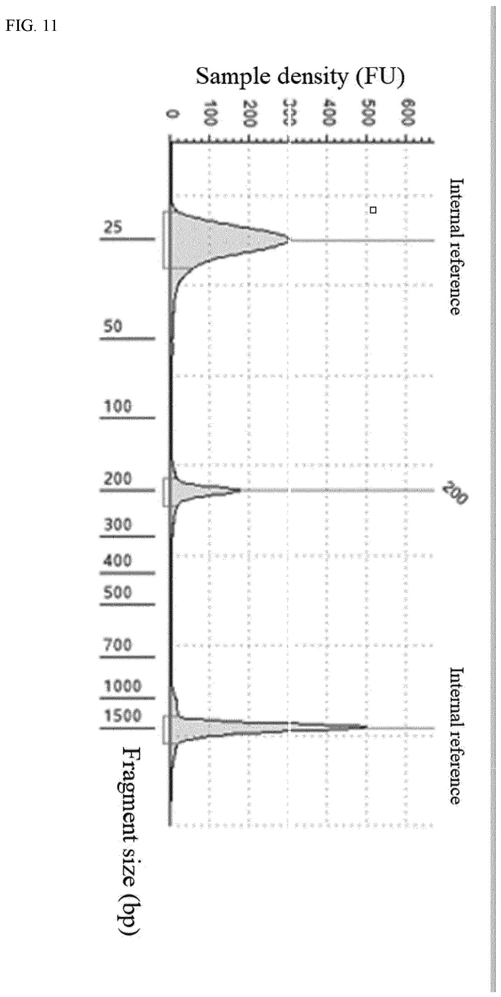

Same steps were performed as those in step 2 of section III of Embodiment 1.
The PCR product was purified and recovered by the magnetic bead (Agencourt AMPure XP, Beckman Coulter, A63880), and detected by Qubit 2.0 and Agilent 2200 TapeStation Systems .
The result of the Agilent 2200 TapeStation Systems detection is shown in FIG. 11.

3. Sequencing and result analysis

The PCR products of all samples were mixed at an equal concentration and diluted to 100 $\mu$M to obtain a DNA library for amplicon sequencing.

The sequencing results are shown in Table 14:

Table 14 shows the comparison of detection results of gene fusion

| Sample No. | Method of the present invention | 63 gene detection |
|---|---|---|
| Patient 1 | EML4-ALK-V3a (E6a A20) | EML4-ALK-V3a (E6a A20) |
| Patient 2 | EML4-ALK-V3b (E6b A20) | EML4-ALK-V3b (E6b A20) |
| Patient 3 | EML4-ALK-V1 (E13 A20) | EML4-ALK-V1 (E13 A20) |
| Healthy subject 1 | None | None |
| Healthy subject 2 | None | None |
| EML4-ALK-V3a (E6a A20) corresponds to EML4_6_ and ALK_20 in Table 11; EML4-ALK-V1 (E13 A20) corresponds to EML4_13_ and ALK_20 in Table 11. | | |

The 63 gene detection product used Agilent's customized probes to perform capture library preparation. The product has been used for detecting thousands of clinical plasma samples, and the performance of the product is stable.

The library prepared by the method of the present invention, when used for sequencing, leads to fusion mutation forms of tested samples consistent with the mutation information of samples obtained by the known 63 gene detection.

The foregoing embodiments are only used to illustrate the present invention. The structure, connection mode, and manufacturing process of each component can be changed. Any equivalent transformation and improvement based on the technical solution of the present invention should not be excluded from the protection scope of the present invention.

Embodiment 5. BRCA1/2 one-step primer pool

I. Design of primers for one-step amplicon sequencing library preparation

The forward outer primers were the same as those in Table 3, others were different, and the barcodes were determined according to the number of samples in the library preparation.

The forward inner primer F2: universal sequence + forward specific primer sequence

The reverse primer R: sequencing adapter 2 + reverse specific primer sequence

Table 15 shows the BRCA1/2 primer set

| Universal sequence | | GGCACCCGAGAATTCCA |
|---|---|---|
| Sequencing adapter 2 | | CCTCTCTATGGGCAGTCGGTGAT |
| | P1_B1_F1 | cacctacctgataccccagatccc |
| | P1_B1_F2 | ccctggagtcgattgattagagccta |
| | P1_B1_F3 | cagttccagtagtcctactttgacact |
| | P1_B1_F4 | tcatcattcacccttggcacagtaa |
| | P1_B1_F5 | taagccttcatccggagagtgta |
| | P1_B1_F6 | cttttataactagattttccttctctccattcc |
| | P1_B1_F7 | ggtccaaagcgagcaagagaatcc |
| | P1_B1_F8 | cgcctggcctgaatgccttaaa |
| Forward specific primer sequence | P1_B1_F9 | aagagcacgttcttctgctgtatg |
| | P1_B1_F10 | gaaatattttctaggaattgcgggagga |
| | P1_B1_F11 | atccagattgatcttgggagtgtaaaaa |
| | P1_B1_F12 | tgtgtgctagaggtaactcatgataatgg |
| | P1_B1_F13 | agaaagggtcaacaaaagaatgtccat |
| | P1_B1_F14 | tgaaagttccccaattgaaagttgcag |

(continued)

| | P1_B1_F15 | gaactttgtaattcaacattcatcgttgtgt |
|---|---|---|
| | P1_B1_F16 | ttagatgataggtggtacatgcacagtt |
| | P1_B1_F17 | taccagtaaaaataaagaaccaggagtgg |
| | P1_B1_F18 | aacctgaattatcactatcagaacaaagca |
| | P1_B1_F19 | tgaacagtacccgttcccttga |
| | P1_B1_F20 | ccttgaggacctgcgaaatccag |
| | P1_B1_F21 | atggaaagcttctcaaagtatttcattttct |
| | P1_B1_F22 | tgcagcgtttatagtctgcttttacatc |
| | P1_B1_F23 | gaacgggcttggaagaaaataatcaag |
| | P1_B1_F24 | ttctgctagcttgttttcttcacagt |
| | P1_B1_F25 | aaacaatataccttctcagtctactaggcat |
| | P1_B1_F26 | gctgtttttagcaaaagcgtccaga |
| | P1_B1_F27 | tcagataacttagaacagcctatgggaag |
| | P1_B1_F28 | gggccaaaattgaatgctatgcttagat |
| | P1_B1_F29 | gagcacaattagccgtaataacattagagaa |
| | P1_B1_F30 | ctggactcattactccaaataaacatgga |
| | P1_B1_F31 | agtctaatatcaagcctgtacagacagtt |
| | P1_B1_F32 | ttgcagaatacattcaaggtttcaaagc |
| | P1_B1_F33 | aaataaatgtgtgagtcagtgtgcag |
| | P1_B1_F34 | ataatgctgaagaccccaaagatctc |
| | P1_B1_F35 | agccaaatgaacagacaagtaaaagaca |
| | P1_B1_F36 | tgcaaattgatagttgttctagcagtgaa |
| | P1_B1_F37 | gcagcagtataagcaatatggaactcgaa |
| | P1_B1_F38 | cggagcagaatggtcaagtgatgaata |
| | P1_B1_F39 | aagagcgtcccctcacaaataaatt |
| | P1_B1_F40 | tgaaagagttcactccaaatcagtagaga |
| | P1_B1_F41 | aggttctgatgactcacatgatggg |
| | P1_B1_F42 | cccctgtgtgagagaaaagaatggaataa |
| | P1_B1_F43 | aaggctgaattctgtaataaaagcaaaca |
| | P1_B1_F44 | cagggtagttctgtttcaaacttgcat |
| | P1_B1_F45 | ttgtatattttcagctgcttgtgaattttct |
| | P1_B1_F46 | tgacagttctgcatacatgtaactagtgt |
| | P1_B1_F47 | ctagttgaatatctgtttttcaacaagtacatttt |
| | P1_B1_F48 | agcggatacaacctcaaaagacg |
| | P1_B1_F49 | gtgtcaagtttctcttcaggaggaaaag |
| | P1_B1_F50 | aaaggaaaataactctcctgaacatctaaaaga |
| | P1_B1_F51 | ttgttgaagagctattgaaaatcatttgtgc |
| | P1_B1_F52 | attatagaggttttctactgttgctgcat |
| | P1_B1_F53 | ggcagttgtgagattatcttttcatggc |
| | P1_B1_F54 | ctctgagaaagaatgaaatggagttgg |

(continued)

| | P1_B2_F1 | aaacaaattttccagcgcttctg |
|---|---|---|
| | P1_B2_F2 | ggtaaaaatgcctattggatccaaaga |
| | P1_B2_F3 | tggtttgaagaactttcttcagaagc |
| | P1_B2_F4 | tcttcttacaactccctatacattctcat |
| | P1_B2_F5 | agtgaaaactaaaatggatcaagcagat |
| | P1_B2_F6 | aaactagtttttgccagttttttaaaataacc |
| | P1_B2_F7 | tttttacccccagtggtatgtg |
| | P1_B2_F8 | tgtacctagcattctgcctcata |
| | P1_B2_F9 | ggatcctgatatgtcttggtcaagtt |
| | P1_B2_F10 | tgaagaagcatctgaaactgtatttcc |
| | P1_B2_F11 | ggactactactatatgtgcattgagagttt |
| | P1_B2_F12 | gaaaacacaaatcaaagagaagctgc |
| | P1_B2_F13 | tggcttataaaatattaatgtgcttctgttt |
| | P1_B2_F14 | aatctacaaaaagtaagaactagcaagac |
| | P1_B2_F15 | aagtgacaaaatctccaaggaagttgt |
| | P1_B2_F16 | gaattctttgccacgtatttctagc |
| | P1_B2_F17 | ggcttcttcatttcagggtatcaaaa |
| | P1_B2_F18 | aatacatactgtttgctcacagaagga |
| | P1_B2_F19 | accgaaagaccaaaaatcagaactaattaa |
| | P1_B2_F20 | tcacagaatgattctgaagaaccaac |
| | P1_B2_F21 | attaccccagaagctgattctctg |
| | P1_B2_F22 | tatatgatcatgaaaatgccagcactc |
| | P1_B2_F23 | ttcccatggaaaagaatcaagatgtat |
| | P1_B2_F24 | actgtcaatccagactctgaagaact |
| | P1_B2_F25 | caggtgataaacaagcaacccaag |
| | P1_B2_F26 | caaatgggcaggactcttagg |
| | P1_B2_F27 | tggcattagataatcaaaagaaactgag |
| | P1_B2_F28 | gaatcaggaagtcagtttgaatttactca |
| | P1_B2_F29 | gcctgttgaaaaatgactgtaacaaaa |
| | P1_B2_F30 | gtgaggaaacttctgcagagg |
| | P1_B2_F31 | tgaagataacaaatatactgctgccag |
| | P1_B2_F32 | aggagggaaacactcagattaaagaag |
| | P1_B2_F33 | tttcagactgcaagtgggaaaaatat |
| | P1_B2_F34 | ccagttggtactggaaatcaactagt |
| | P1_B2_F35 | aaaagagcaaggtactagtgaaatcac |
| | P1_B2_F36 | aaaaaccttgtttctattgagactgtg |
| | P1_B2_F37 | aattcagccttagcttttacacaagt |
| | P1_B2_F38 | tgacaaaaatcatctctccgaaaaaca |
| | P1_B2_F39 | gccagtattgaagaatgttgaagatcaaa |
| | P1_B2_F40 | aataattttgaggtagggccacct |

(continued)

| | P1_B2_F41 | tcataactctctagataatgatgaatgtagc |
|---|---|---|
| | P1_B2_F42 | gtatagggaagcttcataagtcagtct |
| | P1_B2_F43 | agaagatagtaccaagcaagtcttttc |
| | P1_B2_F44 | tagtacagcaagtggaaagcaagt |
| | P1_B2_F45 | ctcagaaatggaaaaaacctgcagtaa |
| | P1_B2_F46 | caggcttcacctaaaaacgtaaaaat |
| | P1_B2_F47 | catgccacacattctcttttacatg |
| | P1_B2_F48 | atataccatacctatagagggagaacagatat |
| | P1_B2_F49 | acattcactgaaaattgtaaagcctataatt |
| | P1_B2_F50 | atatattttctccccattgcagcaca |
| | P1_B2_F51 | aggacatccattttatcaagtttctgc |
| | P1_B2_F52 | tggctctgatgatagtaaaaataagattaatg |
| | P1_B2_F53 | ggttgtgctttttaaatttcaattttatttttgc |
| | P1_B2_F54 | gttccctctgcgtgttctcata |
| | P1_B2_F55 | gctgtatacgtatggcgtttctaaaca |
| | P1_B2_F56 | agttgtagttgttgaattcagtatcatcc |
| | P1_B2_F57 | tgtgcctttcctaaggaatttgctaat |
| | P1_B2_F58 | aaaagataatggaaagggatgacacag |
| | P1_B2_F59 | ctgttaaggcccagttagatcct |
| | P1_B2_F60 | aggcagttctagaagaatgaaaactct |
| | P1_B2_F61 | tagacctttcctctgcccttatc |
| | P1_B2_F62 | cacattattacagtggatggagaagac |
| | P1_B2_F63 | cttctttgggtgttttatgcttggt |
| | P1_B2_F64 | gcagagctttatgaagcagtgaag |
| | P1_B2_F65 | tcttaaatggtcacagggttatttcag |
| | P1_B2_F66 | ggatgtcacaaccgtgtg |
| | P1_B2_F67 | ttccattgcatctttctcatctttct |
| | P1_B1_R1 | atatttagtagccaggacagtagaagg |
| | P1_B1_R2 | gtagagtgctacactgtccaac |
| | P1_B1_R3 | ataaaccaaacccatgcaaaagga |
| | P1_B1_R4 | cccttacagatggagtcttttgg |
| Reverse specific primer sequence | P1_B1_R5 | gatgaaagctccttcaccacaga |
| | P1_B1_R6 | ccactatgtaagacaaaggctgg |
| | P1_B1_R7 | aagaacctgtgtgaaagtatctagca |
| | P1_B1_R8 | gtggtttcttccattgaccacat |
| | P1_B1_R9 | gcattgatggaaggaagcaaatac |
| | P1_B1_R10 | aaagacctttggtaactcagactca |
| | P1_B1_R11 | aaatatttcagtgtccgttcacacaca |
| | P1_B 1_R12 | gcagatgcaaggtattctgtaaag |
| | P1_B1_R13 | acctacataaaactctttccagaatgttg |

(continued)

| | | |
|---|---|---|
| | P1_B1_R14 | ccctttctgttgaagctgtcaatt |
| | P1_B1_R15 | agatggtatgttgccaacacga |
| | P1_B1_R16 | gatgtttccgtcaaatcgtgtg |
| | P1_B1_R17 | agcaataaaagtgtataaatgcctgtatg |
| | P1_B1_R18 | gtagaactatctgcagacacctcaaa |
| | P1_B1_R19 | ccagaaccaccatctttcagtaattt |
| | P1_B1_R20 | atcataaaatgttggagctaggtcct |
| | P1_B1_R21 | tatgatggaagggtagctgttagaag |
| | P1_B1_R22 | ggttaaaatgtcactctgagaggatag |
| | P1_B1_R23 | ggaaatttgtaaaatgtgctccccaa |
| | P1_B1_R24 | aattccttgtcactcagaccaact |
| | P1_B1_R25 | actaaggtgatgttcctgagatg |
| | P1_B1_R26 | ggaagcagggaagctcttcat |
| | P1_B1_R27 | actttccttaatgtcattttcagcaaaac |
| | P1_B1_R28 | cagtctgaactacttcttcatattcttgc |
| | P1_B1_R29 | ctagttctgcttgaatgttttcatcac |
| | P1_B1_R30 | tggaatgttctcatttcccatttctct |
| | P1_B1_R31 | gtttcgttgcctctgaactgaga |
| | P1_B1_R32 | ccttgattttcttccttttgttcacattc |
| | P1_B1_R33 | tttctatgcttgtttcccgactg |
| | P1_B1_R34 | cctagagtgctaacttccagtaac |
| | P1_B1_R35 | cttggaaggctaggattgacaaattc |
| | P1_B1_R36 | ttgttactcttcttggctccagtt |
| | P1_B1_R37 | ttaggtgggcttagatttctactgac |
| | P1_B1_R38 | tgcttataggttcagctttcgtttt |
| | P1_B1_R39 | tccgtttggttagttccctgatttat |
| | P1_B1_R40 | gtattatctgtggctcagtaacaaatg |
| | P1_B1_R41 | ttaaagcctcatgaggatcactg |
| | P1_B1_R42 | agttcatcacttctggaaaaccact |
| | P1_B1_R43 | gggatcagcattcagatctacctttt |
| | P1_B1_R44 | ttcagccttttctacattcattctgtc |
| | P1_B1_R45 | taccctgatactttctggatgcc |
| | P1_B1_R46 | gaatccaaactgatttcatccctgg |
| | P1_B1_R47 | ccagcttcatagacaaaggttctc |
| | P1_B1_R48 | agctgcctaccacaaatacaaattat |
| | P1_B1_R49 | cagagttctcacagttccaaggtta |
| | P1_B1_R50 | gaagaagaagaaaacaaatggttttaccaa |
| | P1_B1_R51 | atcaccacgtcatagaaagtaattgtg |
| | P1_B1_R52 | tcaacaagttgactaaatctcgtactttc |
| | P1_B1_R53 | cattcttacataaaggacactgtgaag |

(continued)

| | P1_B1_R54 | ctctgagaaagaatgaaatggagttgg |
|---|---|---|
| | P1_B2_R1 | ggcatttttacctacgatattcctccaatg |
| | P1_B2_R2 | tgtgacgtactgggtttttagcaag |
| | P1_B2_R3 | gagtcagcccttgctctttgaat |
| | P1_B2_R4 | ttcactgtgcgaagactttttatgtcta |
| | P1_B2_R5 | ggctcttagccaaaatattagcataaaaatcag |
| | P1_B2_R6 | taaaaagcattgttttaatcatacctgactt |
| | P1_B2_R7 | aggtacagatttgtaaatctcagggcaa |
| | P1_B2_R8 | acctcagctcctagactttcagaaatatg |
| | P1_B2_R9 | gatgacaattatcaacctcatctgctctt |
| | P1_B2_R10 | aggtttagagactttctcaaaggcttagat |
| | P1_B2_R11 | tgtgttttcactgtctgtcacagaag |
| | P1_B2_R12 | cgagatcacgggtgacagagc |
| | P1_B2_R13 | aaaaactatcttcttcagaggtatctacaact |
| | P1_B2_R14 | gggcttctgatttgctacatttgaatct |
| | P1_B2_R15 | taggtcttttctgaaatattttggtcacatg |
| | P1_B2_R16 | cagatattgcctgctttactgcaagaa |
| | P1_B2_R17 | atgtatttccagtccactttcagagg |
| | P1_B2_R18 | tttgttttctttttcaaagtggatattaaacct |
| | P1_B2_R19 | acagaaggaatcgtcatctataaaactatatgt |
| | P1_B2_R20 | ctgtagtttttccttattacattttgcttctt |
| | P1_B2_R21 | ctgggattgaaagtcagtatcactgtatt |
| | P1_B2_R22 | tgttacctttgagcttgtctgacattttg |
| | P1_B2_R23 | tttggattactcttagatttgtgttttggttg |
| | P1_B2_R24 | catggtagagttcttgaaaatgggttc |
| | P1_B2_R25 | ggtattttatctatattcaaggagatgtccgatt |
| | P1_B2_R26 | acaatttcaacacaagctaaactagtaggat |
| | P1_B2_R27 | tgccttttggctaggtgttaaattatgg |
| | P1_B2_R28 | tgtctacctgaccaatcgatggg |
| | P1_B2_R29 | cagctttttgcagagcttcagtaga |
| | P1_B2_R30 | ttcaacaaaagtgccagtagtcatttc |
| | P1_B2_R31 | tggccagataatttaagacatatgttgtgc |
| | P1_B2_R32 | tgctccgtttttagtagcagttaactgt |
| | P1_B2_R33 | tgtctgtttcctcataacttagaatgtccat |
| | P1_B2_R34 | ttttcactttgtccaaagattcctttgc |
| | P1_B2_R35 | gagaattctgcatttctttacactttggg |
| | P1_B2_R36 | gggactgatttgtgtaacaagttgcag |
| | P1_B2_R37 | ttcatacaaataatttcctacataatctgcagt |
| | P1_B2_R38 | tcaatactggctcaataccagaatcaagt |
| | P1_B2_R39 | ttttgcagggtgaagagctagtc |

(continued)

| | P1_B2_R40 | caacctgccataattttcgtttggc |
|---|---|---|
| | P1_B2_R41 | tgaagtttccaaactaacatcacaaggtg |
| | P1_B2_R42 | tatttcagaaaacacttgtcttgcgtt |
| | P1_B2_R43 | taccacattatatgaaaagcctttttggg |
| | P1_B2_R44 | gggtttctcttatcaacacgaggaagt |
| | P1_B2_R45 | cccaaaacatgaatgttctcaacaagtg |
| | P1_B2_R46 | tctgtc agttcatcatcttccataaaagc |
| | P1_B2_R47 | tagcataccaagtctactgaataaacacttt |
| | P1_B2_R48 | atgaaatatttctttttaggagaaccctcaa |
| | P1_B2_R49 | acaggtaatcggctctaaagaaacatg |
| | P1_B2_R50 | tgcttgaagatttttccaaagtcagatgt |
| | P1_B2_R51 | tgttttgcttttgtctgttttcctccaa |
| | P1_B2_R52 | aaggcaaaaattcatcacacaaattgtca |
| | P1_B2_R53 | tcagagagattcgaggcagagtg |
| | P1_B2_R54 | cattcctgcactaatgtgttcattct |
| | P1_B2_R55 | atcattggagggtatgagccatcc |
| | P1_B2_R56 | tgccagtttccatatgatccatctatagt |
| | P1_B2_R57 | cagaaaccttaaccatactgccgtatatg |
| | P1_B2_R58 | ggccactttttgggtatctgcacta |
| | P1_B2_R59 | cttcaagaggtgtacaggcatcag |
| | P1_B2_R60 | gggtcaggaaagaatccaagtttggtata |
| | P1_B2_R61 | gaaactccatctcaaacaaacaaacaaattaat |
| | P1_B2_R62 | tcctcctgaattttagtgaataaggcttct |
| | P1_B2_R63 | tgcaaagcacgaacttgctgt |
| | P1_B2_R64 | tgtgatggccagagagtctaaaacag |
| | P1_B2_R65 | gtgacatcccttgataaaccttgttcc |
| | P1_B2_R66 | tagtagtggattttgcttctctgatataaact |
| | P1_B2_R67 | tttttgtcgctgctaactgtatgtta |

II. One-step amplicon sequencing library

1. One-step amplification

The PCR product was obtained using 0.5 pg of gDNA of white blood cell in plasma from healthy subject as a starting sample according to step 1 in section III of Embodiment 1.

2. Magnetic bead purification and Qubit quantification

Same steps were performed as those in step 2 of section III of Embodiment 1.

The PCR product was purified and recovered by the magnetic bead (Agencourt AMPure XP, Beckman Coulter, A63880), and detected by Qubit 2.0 and Agilent 2200 TapeStation Systems .

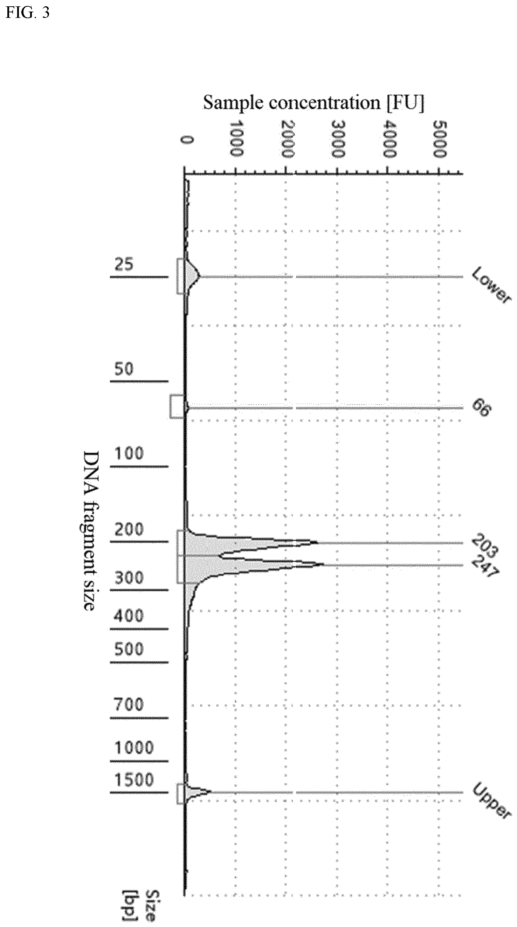

The result of the Agilent 2200 TapeStation Systems detection is shown in FIG. 3. The prepared library is highly specific, and does not have non-specific amplification products or primer dimers. The prepared library has high quality and is suitable for sequencing.

3. Sequencing and result analysis

The PCR products of all samples were mixed at an equal concentration and diluted to 100 μM to obtain a DNA library for amplicon sequencing.

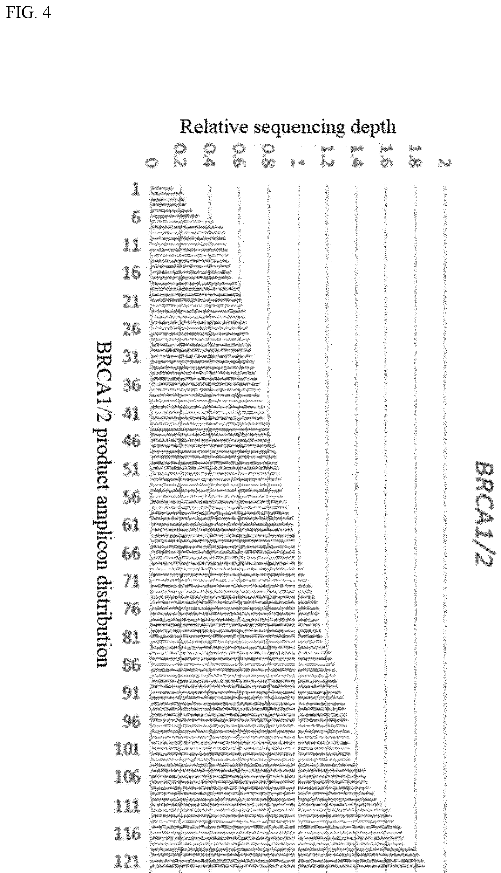

The sequencing results are shown in FIG. 4. The sequencing analysis results show that the 121 amplicons of the BRCA1/2 detection library has a good homogeneity, indicating the advantages of the one-step library preparation technology of the present invention in terms of amplicon homogeneity, and ensuring an effective output of data.

Comparison of three primers used in the method of the comparative example and the method of the present invention and four primers in the prior art

I. Design of primers for one-step amplicon sequencing library preparation

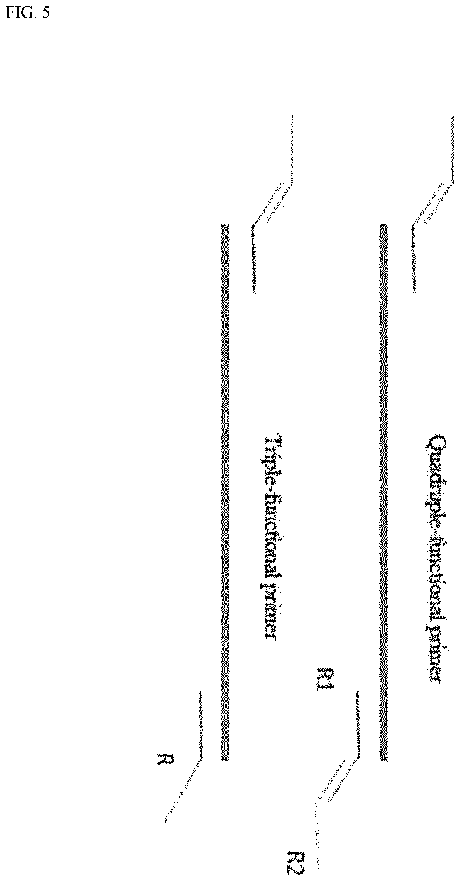

The structures of the 3 primers and the 4 primers designed are shown in FIG. 5.

The present invention:

The 3 primers of the present invention were designed according to the design principle of Embodiment 1:

The forward outer primers were the same as those in Table 3, specifically, there were 67 barcode sequences; the universal sequences were the same as those in Table 15, and the forward specific gene sequences were P1_B2_F1 to P1_B2_F67 in Table 15;
The sequencing adapter 2 was the same as that in Table 15, and the reverse specific primer sequences were P1_B2_R1 to P1_B2_R67 in Table 15;

Control:

The 4 primers were designed according to the following principles in the prior art:

Design principles:

Barcode primer F1: sequencing adapter 1 + barcode sequence + universal sequence 1;
Forward inner primer F2: universal sequence 1 + molecular tag + specific base sequence + forward specific primer sequence;
Reverse outer primer R1: sequencing adapter 2 + universal sequence 2;
Reverse inner primer R2: universal sequence 2 + reverse specific primer sequence;
The sequencing adapter 1 + barcode sequence described above are shown in Table 3.

The rest sequences are shown in Table 16 below:

Table 16 shows the control primer sequences

| | | |
|---|---|---|
| Universal sequence 1 | | GGCACCCGAGAATTCCA |
| Universal sequence 2 | | CCACTACGCCTCCGCTTT |
| Sequencing adapter 2 | | CCTCTCTATGGGCAGTCGGTGAT |
| | P1_B2_F1 | aaacaaattttccagcgcttctg |
| | P1_B2_F2 | ggtaaaaatgcctattggatccaaaga |
| | P1_B2_F3 | tggtttgaagaactttcttcagaagc |
| | P1_B2_F4 | tcttcttacaactccctatacattctcat |
| | P1_B2_F5 | agtgaaaactaaaatggatcaagcagat |
| | P1_B2_F6 | aaactagtttttgccagttttttaaaataacc |
| | P1_B2_F7 | tttttacccccagtggtatgtg |
| | P1_B2_F8 | tgtacctagcattctgcctcata |

(continued)

| | | |
|---|---|---|
| | P1_B2_F9 | ggatcctgatatgtcttggtcaagtt |
| | P1_B2_F10 | tgaagaagcatctgaaactgtatttcc |
| | P1_B2_F11 | ggactactactatatgtgcattgagagttt |
| | P1_B2_F12 | gaaaacacaaatcaaagagaagctgc |
| | P1_B2_F13 | tggcttataaaatattaatgtgcttctgttt |
| | P1_B2_F14 | aatctacaaaaagtaagaactagcaagac |
| | P1_B2_F15 | aagtgacaaaatctccaaggaagttgt |
| | P1_B2_F16 | gaattctttgccacgtatttctagc |
| | P1_B2_F17 | ggcttcttcatttcagggtatcaaaa |
| | P1_B2_F18 | aatacatactgtttgctcacagaagga |
| | P1_B2_F19 | accgaaagaccaaaaatcagaactaattaa |
| | P1_B2_F20 | tcacagaatgattctgaagaaccaac |
| | P1_B2_F21 | attaccccagaagctgattctctg |
| | P1_B2_F22 | tatatgatcatgaaaatgccagcactc |
| | P1_B2_F23 | ttcccatggaaaagaatcaagatgtat |
| | P1_B2_F24 | actgtcaatccagactctgaagaact |
| | P1_B2_F25 | caggtgataaacaagcaacccaag |
| | P1_B2_F26 | caaatgggcaggactcttagg |
| | P1_B2_F27 | tggcattagataatcaaaagaaactgag |
| | P1_B2_F28 | gaatcaggaagtcagtttgaatttactca |
| | P1_B2_F29 | gcctgttgaaaaatgactgtaacaaaa |
| | P1_B2_F30 | gtgaggaaacttctgcagagg |
| | P1_B2_F31 | tgaagataacaaatatactgctgccag |
| | P1_B2_F32 | aggagggaaacactcagattaaagaag |
| | P1_B2_F33 | tttcagactgcaagtgggaaaaatat |
| | P1_B2_F34 | ccagttggtactggaaatcaactagt |
| | P1_B2_F35 | aaaagagcaaggtactagtgaaatcac |
| | P1_B2_F36 | aaaaaccttgtttctattgagactgtg |
| | P1_B2_F37 | aattcagccttagcttttacacaagt |
| | P1_B2_F38 | tgacaaaaatcatctctccgaaaaaca |
| | P1_B2_F39 | gccagtattgaagaatgttgaagatcaaa |
| | P1_B2_F40 | aataattttgaggtagggccacct |
| | P1_B2_F41 | tcataactctctagataatgatgaatgtagc |
| Forward specific primer sequence | P1_B2_F42 | gtatagggaagcttcataagtcagtct |
| | P1_B2_F43 | agaagatagtaccaagcaagtcttttc |
| | P1_B2_F44 | tagtacagcaagtggaaagcaagt |
| | P1_B2_F45 | ctcagaaatggaaaaaacctgcagtaa |
| | P1_B2_F46 | caggcttcacctaaaaacgtaaaaat |
| | P1_B2_F47 | catgccacacattctcttttacatg |
| | P1_B2_F48 | atataccatacctatagagggagaacagatat |

(continued)

| | | |
|---|---|---|
| | P1_B2_F49 | acattcactgaaaattgtaaagcctataatt |
| | P1_B2_F50 | atatattttctccccattgcagcaca |
| | P1_B2_F51 | aggacatccattttatcaagtttctgc |
| | P1_B2_F52 | tggctctgatgatagtaaaaataagattaatg |
| | P1_B2_F53 | ggttgtgctttttaaatttcaattttattttttgc |
| | P1_B2_F54 | gttccctctgcgtgttctcata |
| | P1_B2_F55 | gctgtatacgtatggcgtttctaaaca |
| | P1_B2_F56 | agttgtagttgttgaattcagtatcatcc |
| | P1_B2_F57 | tgtgcctttcctaaggaatttgctaat |
| | P1_B2_F58 | aaaagataatggaaagggatgacacag |
| | P1_B2_F59 | ctgttaaggcccagttagatcct |
| | P1_B2_F60 | aggcagttctagaagaatgaaaactct |
| | P1_B2_F61 | tagaccttttcctctgcccttatc |
| | P1_B2_F62 | cacattattacagtggatggagaagac |
| | P1_B2_F63 | cttctttgggtgttttatgcttggt |
| | P1_B2_F64 | gcagagctttatgaagcagtgaag |
| | P1_B2_F65 | tcttaaatggtcacagggttatttcag |
| | P1_B2_F66 | ggatgtcacaaccgtgtg |
| | P1_B2_F67 | ttccattgcatctttctcatctttct |
| | P1_B2_R1 | ggcatttttacctacgatattcctccaatg |
| | P1_B2_R2 | tgtgacgtactgggtttttagcaag |
| | P1_B2_R3 | gagtcagcccttgctctttgaat |
| | P1_B2_R4 | ttcactgtgcgaagacttttatgtcta |
| | P1_B2_R5 | ggctcttagccaaaatattagcataaaaatcag |
| | P1_B2_R6 | taaaaagcattgttttaatcatacctgactt |
| | P1_B2_R7 | aggtacagatttgtaaatctcagggcaa |
| | P1_B2_R8 | acctcagctcctagactttcagaaatatg |
| | P1_B2_R9 | gatgacaattatcaacctcatctgctctt |
| | P1_B2_R10 | aggtttagagactttctcaaaggcttagat |
| | P1_B2_R11 | tgtgttttcactgtctgtcacagaag |
| | P1_B2_R12 | cgagatcacgggtgacagagc |
| | P1_B2_R13 | aaaaactatcttcttcagaggtatctacaact |
| | P1_B2_R14 | gggcttctgatttgctacatttgaatct |
| | P1_B2_R15 | taggtcttttctgaaatattttggtcacatg |
| | P1_B2_R16 | cagatattgcctgctttactgcaagaa |
| | P1_B2_R17 | atgtatttccagtccactttcagagg |
| Reverse specific primer sequence | P1_B2_R18 | tttgttttcttttcaaagtggatattaaacct |
| | P1_B2_R19 | acagaaggaatcgtcatctataaaaactatatgt |
| | P1_B2_R20 | ctgtagttttccttattacattttgcttctt |
| | P1_B2_R21 | ctgggattgaaagtcagtatcactgtatt |

(continued)

| | P1_B2_R22 | tgttacctttgagcttgtctgacattttg |
|---|---|---|
| | P1_B2_R23 | tttggattactcttagatttgtgttttggttg |
| | P1_B2_R24 | catggtagagttcttgaaaatgggttc |
| | P1_B2_R25 | ggtattttatctatattcaaggagatgtccgatt |
| | P1_B2_R26 | acaatttcaacacaagctaaactagtaggat |
| | P1_B2_R27 | tgccttttggctaggtgttaaattatgg |
| | P1_B2_R28 | tgtctacctgaccaatcgatggg |
| | P1_B2_R29 | cagctttttgcagagcttcagtaga |
| | P1_B2_R30 | ttcaacaaaagtgccagtagtcatttc |
| | P1_B2_R31 | tggccagataatttaagacatatgttgtgc |
| | P1_B2_R32 | tgctccgttttagtagcagttaactgt |
| | P1_B2_R33 | tgtctgtttcctcataacttagaatgtccat |
| | P1_B2_R34 | tttttcactttgtcc aaagattcctttgc |
| | P1_B2_R35 | gagaattctgcatttctttacactttggg |
| | P1_B2_R36 | gggactgatttgtgtaacaagttgcag |
| | P1_B2_R37 | ttcatacaaataatttcctacataatctgcagt |
| | P1_B2_R38 | tcaatactggctcaataccagaatcaagt |
| | P1_B2_R39 | tttttgcagggtgaagagctagtc |
| | P1_B2_R40 | caacctgccataattttcgtttggc |
| | P1_B2_R41 | tgaagtttccaaactaacatcacaaggtg |
| | P1_B2_R42 | tatttcagaaaacacttgtcttgcgtt |
| | P1_B2_R43 | taccacattatatgaaaagccttttttggg |
| | P1_B2_R44 | gggtttctcttatcaacacgaggaagt |
| | P1_B2_R45 | cccaaaacatgaatgttctcaacaagtg |
| | P1_B2_R46 | tctgtcagttcatcatcttccataaaagc |
| | P1_B2_R47 | tagcataccaagtctactgaataaacacttt |
| | P1_B2_R48 | atgaaatatttctttttaggagaaccctcaa |
| | P1_B2_R49 | acaggtaatcggctctaaagaaacatg |
| | P1_B2_R50 | tgcttgaagatttttccaaagtcagatgt |
| | P1_B2_R51 | tgttttgcttttgtctgtttttcctccaa |
| | P1_B2_R52 | aaggcaaaaattcatcacacaaattgtca |
| | P1_B2_R53 | tcagagagattcgaggcagagtg |
| | P1_B2_R54 | cattcctgcactaatgtgttcattct |
| | P1_B2_R55 | atcattggagggtatgagccatcc |
| | P1_B2_R56 | tgccagtttccatatgatccatctatagt |
| | P1_B2_R57 | cagaaaccttaaccatactgccgtatatg |
| | P1_B2_R58 | ggccactttttgggtatctgcacta |
| | P1_B2_R59 | cttcaagaggtgtacaggcatcag |
| | P1_B2_R60 | gggtcaggaaagaatccaagtttggtata |
| | P1_B2_R61 | gaaactccatctcaaacaaacaaacaaattaat |

(continued)

| | | |
|---|---|---|
| | P1_B2_R62 | tcctcctgaattttagtgaataaggcttct |
| | P1_B2_R63 | tgcaaagcacgaacttgctgt |
| | P1_B2_R64 | tgtgatggccagagagtctaaaacag |
| | P1_B2_R65 | gtgacatcccttgataaaccttgttcc |
| | P1_B2_R66 | tagtagtggattttgcttctctgatataaact |
| | P1_B2_R67 | tttttttgtcgctgctaactgtatgtta |

II. One-step amplicon sequencing library

The method was the same as that in step 2 of Embodiment 2.

The sequencing results are analyzed as follows:

1. The homogeneity results of the libraries prepared by triple-functional component primer pool and quadruple-functional component primer pool

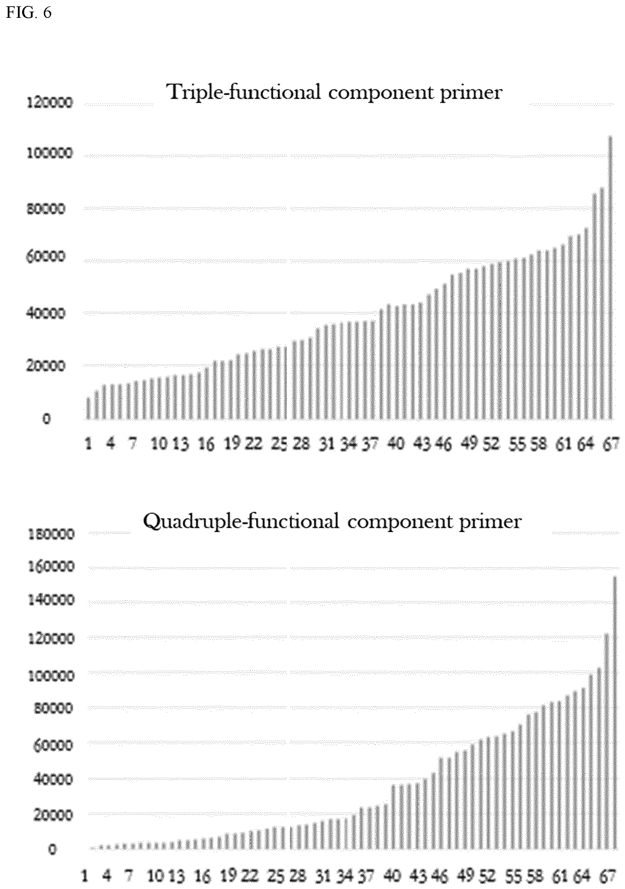

The homogeneity of the amplicons library is a very important indicator of the quality of the library. Good homogeneity of the library indicates a higher coverage of the target region of the library, and a better detection accuracy of the panel covering region. For this purpose, under the premise of ensuring the intact functional structure of the primer, the primer design of the amplicon is improved. The improved primer structure is optimized and simplified from the original F1+F2+R1+R2 (quadruple-functional primer components) to F1+F2+R (triple-functional primer components). This design will increase the stability of the reaction system and ensure the homogeneity of amplicons in the library.
Amplifications were respectively carried out on the primer set of the present invention and the control primer set using the same white blood cell DNA sample as a template.
The results are shown in FIG. 6. When the ratio of the specific primer is not adjusted, the comparison between the homogeneity of amplicons in the library prepared by triple-functional primer components and the homogeneity of amplicons in the library prepared by quadruple-functional primer components of the library of 67 amplicons (67 pairs of amplicons of BRCA2 selected from the 121 pairs of primers in Embodiment 5) indicates that the triple-functional component primer has significant advantages in the homogeneity of the library.

2. 30 ng of cfDNA was used in a library preparation by one-step primer pool, and the number of molecular tag types/ the number of clusters of one of the amplicons was obtained after data analysis

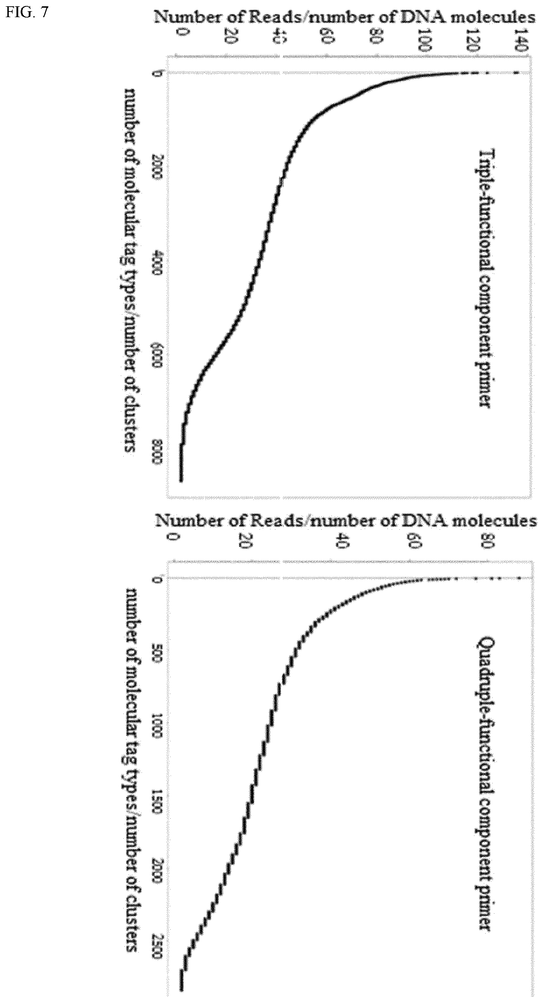

Amplifications were respectively carried out on the primer set of the present invention and the control primer set using the same cfDNA sample as a template.
The results are shown in FIG. 7. Compared with the quadruple-functional component primer, the triple-functional component primer has better capture efficiency of original template than the quadruple-functional component primer, which makes the ultra-low frequency detection more sensitive and stable. The figure below is an amplicon randomly selected in the triple-functional component primer method, and after library preparation, the data information after adding tags to the original template is obtained. The higher template capture efficiency allows the triple-functional component primer method to reach a lower detection limit of mutation frequency.

3. Background noise at the level of 0.1‰-1‰ of the libraries prepared by two methods and subjected to sequencing (same as 2)

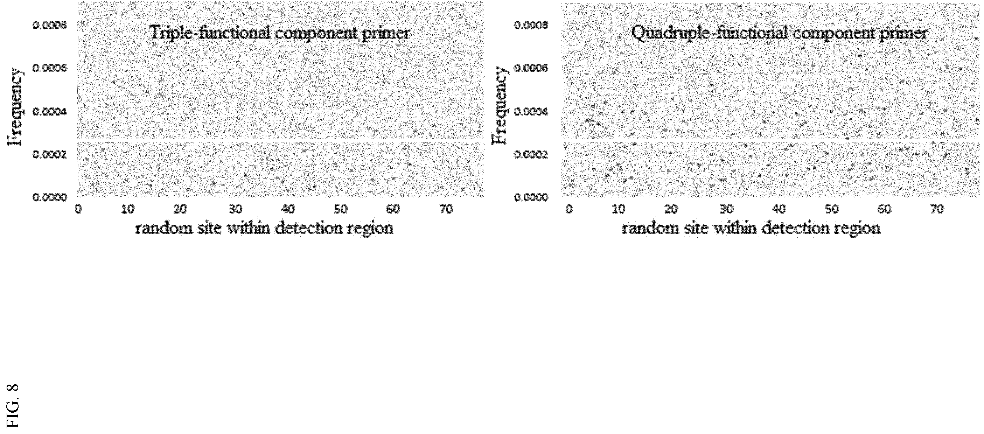

Amplifications were respectively carried out on the primer set of the present invention and the control primer set using the same cfDNA sample as a template.
The results are shown in FIG. 8. Compared with the amplicon library preparation method of the quadruple-functional component primer, using the triple-functional component primer effectively improves the capture efficiency of the template, reduces the non-specific amplification of the library, and decreases the number of cycles of the library amplification. At the same time, through the comparison of two amplicon library preparation methods, it is found that under the use of high-fidelity DNA polymerase, the triple-functional component primer is better in terms of the background noise of sequencing data at the level of $5\text{‰}$. The lower background noise enables the triple-functional primer component method to be more accurate in detecting a relatively low frequency mutation.

Good amplification homogeneity, high capture efficiency of original template molecules, high-fidelity DNA polymerase, ultra-low background noise, and the introduction of molecular tags eventually facilitate the triple-functional primer component to achieve an effective detection of ultra-low frequency mutation at the level of $3\%_{00}$. The primer structure of this library preparation method has been fully optimized, and the performance of this library preparation method is much better than that of the traditional library preparation method for low-frequency mutation detection.

The comparison results of the one-step rapid library preparation method of the present invention, the ordinary amplification library preparation method and the capture library preparation method are shown in Table 17 and Table 18.

Table 17 shows the comparison of the one-step rapid library preparation method, the ordinary amplification library preparation method, and the capture library preparation method

|  | One-step rapid library preparation | Ordinary amplification library preparation | Capture library preparation |
|---|---|---|---|
| Sample required | Very little | little | Much (100-500 ng) |
| Sample capture efficiency | Very high | high | Relatively high |
| operation | <5 min | Relatively complicated | Very complicated |
| Library preparation time | < 1.5 h | 8 h | 2 d |
| Contamination risk | Extremely low risk of cross-contamination | Potential contamination risk | Potential contamination risk |
| Laboratory requirement | Low | Relatively high | High |
| Quantification method | Qubit quantification | qPCR quantification | qPCR quantification |
| Flexibility | Good | Good | Poor (difficult to increase or decrease capture region) |
| Capture region | Moderate | Moderate | Very large |
| Library preparation cost | Very low | Relatively high | Very high |
| Operator requirement | Low | Relatively high | Very high |

Table 18 shows the comparison results of the proportion of target fragments in the library of the present method and the control method

| Sample No | RIN | Proportion of main peak of the one-step library | Proportion of main peak of the control library |
|---|---|---|---|
| LAAAFST1 | 3.2 | 50.66% | 30.28% |
| PC949TQ2 | 2.9 | 100% | 57.48% |
| PA970TQ1 | 2.8 | 100% | 56.71% |
| LAAAF0T1 | 2.8 | 100% | 73.34% |
| LAAAFPT 1 | 2.7 | 84.86% | 59.94% |
| PD010TQ1 | 2.3 | 80.17% | 69.85% |
| PC916TQ1 | 2.2 | 100% | 82.54% |
| PC980TQ 1 | 2 | 100% | 52.30% |
| PC977TP1 | 1.7 | 100% | 51.83% |
| LAAAEVT1 | 1.7 | 100% | 38.01% |

After the amplicon library is prepared, there may be amplification products of target fragments, primer dimers or multimers, and fragment products of non-specific amplification in the system. A high proportion of the amplification products of target fragments becomes an extremely important indicator for evaluating the quality of the amplicon library. Table 18 shows the present method has great advantages in terms of the proportion of target fragments of the library as compared to the control method.

**Industrial application**

In order to solve the current difficulties in library preparation, the present invention has developed the one-step rapid amplification library preparation method. Compared with the traditional capture method, the amplification library preparation method has the following advantages (FIG. 1). The library preparation method is simple and rapid, has a low requirement for operators, and can achieve the library preparation by only a normal PCR operation for corresponding reaction time. Since the quality and purity of the library prepared by this method are very high, only a simple cycle of magnetic bead purification and Qubit quantification are required before being used in a normal sequencing. The one-step library preparation technology can be applied to all second-generation platforms including IonTorrent, illumina and BGI/MGI platforms. Based on the library preparation method, the present invention has developed detection products targeted at SNP, Ins/Del, CNV and methylation of DNA, as well as detection products for gene fusion and expression of RNA samples.

The present invention has the following merits because of adopting the above technical solutions:

1. Little sample consumption and high utilization rate. The capture efficiency of the original template molecules in the sample is high, and thus a relatively low amount of starting templates is required. When performing germline mutation detection, even just a pg-level amount of starting templates is required. When performing low frequency mutation detection of cfDNA, a limited amount of starting templates can achieve a higher template capture efficiency, thereby achieving an effective capture of trace ctDNA molecules, realizing a lower detection limit and a higher sensitivity;

2. Ultra-low detection limit. The unique primer design, supporting PCR reaction system, reaction conditions, and subsequent information analysis and noise reduction system ultimately result in the lowest mutation detection limit of 3 $‰$, making it possible to realize an accurate detection of ultra-early stage and trace amounts of ctDNA sample mutations;

3. Good homogeneity of library. The innovative primer structure design and supporting reaction system result in the optimal homogeneity of amplicons in the library. When conducting a multiplex amplification, the different structural characteristics of the sequences of various amplicons and the different amplification efficiencies of various primers will eventually result in a huge difference in the abundance of amplicons in the library. How to balance the difference in the abundance of amplicons is a key indicator to evaluate the quality of the library. The components of the triple-functional primer used in the present method have obvious advantages over the components of the quadruple-functional primer. Specifically, the cooperation of primer composition and reaction system ensures that the method can control differential amplifications of amplicons at a reduced number of cycles, and then a method like universal primer amplification is used. Since there is no competition between R1 and R2 in the following figure, a stable low differential amplification is achieved in subsequent cycles;

4. High repeatability. The components of the quadruple-functional primer will increase the uncertainty of the reaction system and reaction conditions, and are more sensitive to sample quality, reaction system and external environmental influences. While the components of the triple-functional primer have been improved in this aspect, and the simpler components result in a better system stability, and a higher repeatability and accuracy of sample detection;

5. Easy operation and time saving. The traditional capture library preparation technology has cumbersome operations and long procedures. The entire library preparation process takes nearly 48 h and imposes high requirements on operators. The ordinary amplification library preparation method requires at least two cycles of PCR and two cycles of purification, including subsequent QPCR quantification. The entire library preparation process requires at least one working day. The present invention only involves one-step PCR reaction and corresponding product purification steps, and the entire library preparation process can be completed within 1.5 h, thereby simplifying the library preparation operation process and saving time of the library preparation (the library preparation can be completed within 1.5 h, and the entire process from the library preparation to the completion of sequencing and to the completion of the bioinformatic analysis can be controlled within 22 h);

6. Able to detect multiple gene mutation types. Starting from a DNA sample, SNP, SNV, Ins/Del, methylation, gene or exon level copy number variation, and chromosome arm level copy number variation can be detected. In addition, after adding molecular tags to primers, mutations at the level of as low as 1‰ can be further detected. Starting with a RNA sample, the expression of specific genes, the fusion of specific genes, etc. can be detected;

7. Multiple sample types. The starting sample can be fresh tissue samples, frozen samples, puncture samples, FFPE samples and other tissue sample types. Meanwhile, isolated cfDNA or CTC in blood, urine, cerebrospinal fluid, and pleural fluid can also be detected. After DNA or RNA is extracted from normal samples, library preparation can be conducted by one-step rapid amplification library preparation method;

8. Effective elimination of cross-contamination between samples. The barcode sequences that distinguish different samples are added at the beginning of PCR, and the simplification of the operation process and steps effectively eliminates possible cross-contaminations during the library preparation process, especially when detecting low frequency mutations, cross-contamination between samples is extremely prone to determining as a false positive mutation;

9. Reduced cost of library preparation. Compared with the traditional capture technology, the cost required for library preparation using the present method is greatly reduced. The capture probes used in the traditional capture library preparation are expensive, and the reagents and consumables involved in the lengthy experimental process also increase the cost of capture library preparation. In contrast, the one-step library preparation process requires a greatly reduced amount of reagents and consumables, and the cost of library preparation is much lower than that of the traditional capture library preparation method. At the same time, compared with the one-step rapid amplification library preparation method, at least one cycle of additional PCR and purification and the QPCR quantitation of the library in the normal amplification library preparation method will also greatly increase the cost of library preparation. Compared with the components of the prior quadruple-functional primer, the components of the triple-functional primer lead to low consumption of total primer and each component of primer, thus having a lower cost advantage;

10. Space saving. Since this method requires only one cycle of PCR, the laboratory requires only 3 rooms (sample extraction, PCR amplification room, library purification and sequencing), which saves space as compared to the conventional library preparation where 4 rooms (sample extraction, PCR1, PCR2, and library purification and sequencing) are required.

Flexible and simple library preparation method, allowing detection of multiple mutation types, and extremely high detection sensitivity are the biggest features of the present invention.

## Claims

1. A primer combination for preparing an amplicon library for detecting the variation of a target gene, consisting of:

   a forward outer primer F1, a forward inner primer F2, and a reverse primer R designed according to a target amplicon; wherein
   the forward outer primer F1 is sequentially composed of a sequencing adapter 1, a barcode sequence for distinguishing different samples, and a universal sequence;
   the forward inner primer F2 is sequentially composed of a universal sequence and a forward specific primer sequence of the target amplicon, wherein the forward outer primer F1 and the forward inner primer F2 share a normal universal sequence;
   the reverse outer primer R is sequentially composed of a sequencing adapter 2 and a reverse specific primer sequence of the target amplicon.

2. The primer combination according to claim 1, wherein the forward inner primer F2 is sequentially composed of the universal sequence, a molecular tag sequence, and the forward specific primer sequence of the target amplicon.

3. The primer combination according to claim 2, wherein the molecular tag sequence is composed of 6-30 bases, comprising random bases and at least one set of specific bases; each set of specific bases is among the random bases; the specific bases of each set are composed of 1-5 bases.

4. The primer combination according to any one of claims 1-3, wherein the barcode sequence is a nucleotide sequence with a length of 6-12 nt, no more than 3 consecutive bases, and a GC content of 40-60%;
   the universal sequence has a length of 16-25 nt, and a GC content of 35-65%, without consecutive bases and secondary structure.

5. The primer combination according to any one of claims 1-3, wherein the sequencing adapter 1 and the sequencing adapter 2 are corresponding sequencing adapters selected according to different sequencing platforms.

6. The primer combination according to claim 5, wherein:

   When the sequencing platform is an Illumina platform, the sequencing adapter 1 is I5, and the sequencing adapter 2 is I7, wherein the I5 adapter and I7 adapter are complementary to the primer sequences on the chip;

or the sequencing platform is an Ion Torrent platform, the sequencing adapter 1 is A, and the sequencing adapter 2 is P, wherein the A adapter is used for sequencing and complementary to the sequencing primer, and the P adapter is complementary to the sequence on the vector, so as to link a template to the vector;

or the sequencing platform is a BGI/MGI platform;

or, the nucleotide sequence of the universal sequence is shown in SEQ ID NO: 1.

7. A kit for preparing an amplicon library for detecting the variation of a target gene, comprising the primer combination according to any one of claims 1-6.

8. Use of the primer combination according to any one of claims 1-6 or the kit according to claim 7,

(1) for preparing the amplicon library for detecting the variation of the target gene;
(2) for detecting mutation sites or variations in a target region of a sample to be tested; or
(3) for detecting the variation frequency of the target region of the sample to be tested.

9. A method of preparing an amplicon library for detecting the variation of a target gene, comprising the following steps: taking DNA or cDNA of a sample to be tested as a template, carrying out a one-step PCR amplification using the primer combination according to any one of claims 1-6 or the kit according to claim 7 to obtain an amplified product, wherein the amplified product is the amplicon library of the target gene.

10. The method according to claim 9, wherein the sample to be tested is an in vitro tissue sample, a frozen sample, a puncture sample, a FFPE sample, blood, urine, cerebrospinal fluid, or pleural fluid.

11. A method of detecting a mutation of a target gene of a sample to be tested, comprising the following steps:

1) preparing an amplicon library of the target gene by the method according to claim 9;
2) evenly mixing the amplicon libraries of the target genes of all samples, and then diluting to obtain a sequencing DNA library;
3) sequencing the sequencing DNA library to obtain a sequencing result, and analyzing the variation of the target gene of the sample to be tested according to the sequencing result.

12. The method according to claim 11, wherein the sample to be tested is an in vitro tissue sample, a frozen sample, a puncture sample, a FFPE sample, blood, urine, cerebrospinal fluid, or pleural fluid.

13. A method of detecting a mutation frequency in a target region of a sample to be tested, comprising the following steps:

1) preparing an amplicon library of the target gene by using the method according to claim 9;
2) evenly mixing the amplicon libraries of the target genes of all samples, and then diluting to obtain a sequencing DNA library;
3) sequencing the sequencing DNA library to obtain a sequencing result, and calculating the mutation frequency of the target gene of the sample to be tested according to the sequencing result;

wherein the variation frequency=number of mutation clusters/total number of effective clusters×100%.

14. The method according to claim 13, wherein the sample to be tested is an in vitro tissue sample, a frozen sample, a puncture sample, a FFPE sample, blood, urine, cerebrospinal fluid, or pleural fluid.

**Patentansprüche**

1. Primerkombination zum Erstellen einer Amplikonbibliothek, um die Variation eines Zielgens zu erkennen, bestehend aus:

einem äußeren Vorwärtsprimer F1, einem inneren Vorwärtsprimer F2 und einem Rückwärtsprimer R, die entsprechend einem Zielamplikon ausgestaltet sind; wobei
der äußere Vorwärtsprimer F1 nacheinander aus einem Sequenzierungsadapter 1, einer Strichcode-Sequenz zur Unterscheidung verschiedener Proben und einer Universalsequenz aufgebaut ist;

der innere Vorwärtsprimer F2 nacheinander aus einer Universalsequenz und einer spezifischen Vorwärtsprimersequenz des Zielamplikons aufgebaut ist, wobei der äußere Vorwärtsprimer F1 und der innere Vorwärtsprimer F2 eine normale Universalsequenz gemeinsam haben;

der äußere Rückwärtsprimer R nacheinander aus einem Sequenzierungsadapter 2 und einer spezifischen Rückwärtsprimersequenz des Zielamplikons aufgebaut ist.

2. Primerkombination nach Anspruch 1, wobei der innere Vorwärtsprimer F2 nacheinander aus der Universalsequenz, einer Molekül-Tagsequenz und der spezifischen Vorwärtsprimersequenz des Zielamplikons aufgebaut ist.

3. Primerkombination nach Anspruch 2, wobei die Molekül-Tagsequenz aus 6-30 Basen zusammengesetzt ist, die willkürliche Basen und mindestens einen Satz spezifischer Basen umfassen; wobei jeder Satz spezifischer Basen Teil der willkürlichen Basen ist, wobei die spezifischen Basen von jedem Satz aus 1-5 Basen zusammengesetzt sind.

4. Primerkombination nach einem der Ansprüche 1-3, wobei die Strichcodesequenz eine Nukleotidsequenz mit einer Länge von 6-12 nt, nicht mehr als 3 aufeinanderfolgenden Basen und einem GC-Gehalt von 40-60% ist; die Universalsequenz eine Länge von 16-25 nt und einen GC-Gehalt von 35-65% aufweist, ohne aufeinanderfolgende Basen und Sekundärstruktur.

5. Primerkombination nach einem der Ansprüche 1-3, wobei der Sequenzierungsadapter 1 und der Sequenzierungsadapter 2 entsprechende Sequenzierungsadapter sind, die je nach unterschiedlichen Sequenzierungsplattformen ausgewählt sind.

6. Primerkombination nach Anspruch 5, wobei:

wenn die Sequenzierungsplattform eine Illumina-Plattform ist, I5 der Sequenzierungsadapter 1 ist und I7 der Sequenzierungsadapter 2 ist, wobei der Adapter I5 und der Adapter I7 komplementär zu den Primersequenzen auf dem Chip sind;

oder die Sequenzierungsplattform eine Ion-Torrent-Plattform ist, A der Sequenzierungsadapter 1 ist und P der Sequenzierungsadapter 2 ist, wobei der Adapter A zum Sequenzieren genutzt wird und komplementär zum Sequenzierungsprimer ist und der Adapter P komplementär zu der Sequenz auf dem Vektor ist, damit ein Templat mit dem Vektor verbunden wird;

oder die Sequenzierungsplattform eine BGI/MGI-Plattform ist;

oder die Nukleotidsequenz der Universalsequenz in SEQ ID-Nr. 1 dargestellt ist.

7. Kit zum Erstellen einer Amplikonbibliothek, um die Variation eines Zielgens zu erkennen, umfassend die Primerkombination nach einem der Ansprüche 1-6.

8. Verwendung der Primerkombination nach einem der Ansprüche 1-6 oder des Kits nach Anspruch 7

(1) zum Erstellen der Amplikonbibliothek, um die Variation des Zielgens zu erkennen;

(2) zum Erkennen von Mutationsstellen oder Variationen in einem Zielbereich einer zu testenden Probe; oder

(3) zum Erkennen der Variationshäufigkeit des Zielbereichs der zu testenden Probe.

9. Verfahren zum Erstellen einer Amplikonbibliothek, um die Variation eines Zielgens zu erkennen, umfassend die folgenden Schritte:

Verwenden von DNA oder cDNA einer zu testenden Probe als Templat, Durchführen einer One-Step-PCR-Vervielfältigung unter Verwendung der Primerkombination nach einem der Ansprüche 1-6 oder des Kits nach Anspruch 7 zum Erhalten eines vervielfältigten Produkts, wobei das vervielfältigte Produkt die Amplikonbibliothek des Zielgens ist.

10. Verfahren nach Anspruch 9, wobei die zu testende Probe eine In-vitro-Gewebeprobe, eine gefrorene Probe, eine mittels Punktion erhaltene Probe, eine FFPE-Probe, Blut, Urin, Cerebrospinalflüssigkeit oder Pleuraflüssigkeit ist.

11. Verfahren zum Erkennen einer Mutation eines Zielgens in einer zu testenden Probe, umfassend die folgenden Schritte:

1) Erstellen einer Amplikonbibliothek des Zielgens mit dem Verfahren nach Anspruch 9;

2) gleichmäßiges Mischen der Amplikonbibliotheken der Zielgene sämtlicher Proben und dann Verdünnen, um eine Sequenzierungs-DNA-Bibliothek zu erhalten;
3) Sequenzieren der Sequenzierungs-DNA-Bibliothek zum Erhalten eines Sequenzierungsergebnisses und Analysieren der Variation des Zielgens der zu testenden Probe je nach dem Sequenzierungsergebnis.

**12.** Verfahren nach Anspruch 11, wobei die zu testende Probe eine In-vitro-Gewebeprobe, eine gefrorene Probe, eine mittels Punktion erhaltene Probe, eine FFPE-Probe, Blut, Urin, Cerebrospinalflüssigkeit oder Pleuraflüssigkeit ist.

**13.** Verfahren zum Erkennen einer Mutationshäufigkeit in einem Zielbereich in einer zu testenden Probe, umfassend die folgenden Schritte:

1) Erstellen einer Amplikonbibliothek des Zielgens unter Verwendung des Verfahrens nach Anspruch 9;
2) gleichmäßiges Mischen der Amplikonbibliotheken der Zielgene sämtlicher Proben und dann Verdünnen, um eine Sequenzierungs-DNA-Bibliothek zu erhalten;
3) Sequenzieren der Sequenzierungs-DNA-Bibliothek zum Erhalten eines Sequenzierungsergebnisses und Berechnen der Mutationshäufigkeit des Zielgens der zu testenden Probe je nach dem Sequenzierungsergebnis;

wobei die Variationshäufigkeit = Anzahl von Mutationsclustern/Gesamtzahl effektiver Cluster $\times$ 100%.

**14.** Verfahren nach Anspruch 13, wobei die zu testende Probe eine In-vitro-Gewebeprobe, eine gefrorene Probe, eine mittels Punktion erhaltene Probe, eine FFPE-Probe, Blut, Urin, Cerebrospinalflüssigkeit oder Pleuraflüssigkeit ist.

**Revendications**

**1.** Combinaison d'amorces destinée à la préparation d'une banque d'amplicons pour détecter la variation d'un gène cible, constituée de :

une amorce sens externe F1, une amorce sens interne F2, et une amorce antisens R conçues selon un amplicon cible ; dans laquelle
l'amorce sens externe F1 est composée de manière séquentielle d'un adaptateur de séquençage 1, d'une séquence de signature pour distinguer différents échantillons, et d'une séquence universelle ;
l'amorce sens interne F2 est composée de manière séquentielle d'une séquence universelle et d'une séquence d'amorce spécifique sens de l'amplicon cible, l'amorce sens externe F1 et l'amorce sens interne F2 partageant une séquence universelle normale ;
l'amorce antisens externe R est composée de manière séquentielle d'un adaptateur de séquençage 2 et d'une séquence d'amorce spécifique antisens de l'amplicon cible.

**2.** Combinaison d'amorces selon la revendication 1, dans laquelle l'amorce sens interne F2 est composée de manière séquentielle de la séquence universelle, d'une séquence d'étiquette moléculaire, et de la séquence d'amorce spécifique sens de l'amplicon cible.

**3.** Combinaison d'amorces selon la revendication 2, dans laquelle la séquence d'étiquette moléculaire est composée de 6 à 30 bases, comprenant des bases aléatoires et au moins un jeu de bases spécifiques ; chaque jeu de bases spécifiques se trouvant parmi les bases aléatoires ; les bases spécifiques de chaque jeu étant composées de 1 à 5 bases.

**4.** Combinaison d'amorces selon l'une quelconque des revendications 1 à 3, dans laquelle la séquence de signature est une séquence nucléotidique ayant une longueur de 6 à 12 nt, pas plus de 3 bases consécutives, et un taux de GC de 40 à 60 % ;
la séquence universelle présente une longueur de 16 à 25 nt et un taux de GC de 35 à 65 %, sans bases consécutives ni structure secondaire.

**5.** Combinaison d'amorces selon l'une quelconque des revendications 1 à 3, dans laquelle l'adaptateur de séquençage 1 et l'adaptateur de séquençage 2 sont des adaptateurs de séquençage correspondants choisis selon différentes plateformes de séquençage.

**6.** Combinaison d'amorces selon la revendication 5, dans laquelle :

lorsque la plateforme de séquençage est une plateforme Illumina, l'adaptateur de séquençage 1 est I5 et l'adaptateur de séquençage 2 est I7, l'adaptateur I5 et l'adaptateur I7 étant complémentaires des séquences d'amorce sur la puce ;

ou que la plateforme de séquençage est une plateforme Ion Torrent, l'adaptateur de séquençage 1 est A et l'adaptateur de séquençage 2 est P, l'adaptateur A étant utilisé pour le séquençage et en complément de l'amorce de séquençage et l'adaptateur P étant complémentaire de la séquence sur le vecteur de façon à lier un modèle au vecteur ;

ou la plateforme de séquençage est une plateforme BGI/MGI ;

ou la séquence nucléotidique de la séquence universelle est indiquée dans SEQ ID NO : 1.

7. Kit de préparation d'une banque d'amplicons pour détecter la variation d'un gène cible, comprenant la combinaison d'amorces selon l'une quelconque des revendications 1 à 6.

8. Utilisation de la combinaison d'amorces selon l'une quelconque des revendications 1 à 6 ou du kit selon la revendication 7,

(1) pour préparer la banque d'amplicons pour détecter la variation du gène cible ;
(2) pour détecter des sites de mutation ou des variations dans une région cible d'un échantillon à tester ; ou
(3) pour détecter la fréquence de variation de la région cible de l'échantillon à tester.

9. Procédé de préparation d'une banque d'amplicons pour détecter la variation d'un gène cible, comprenant les étapes suivantes :
prendre de l'ADN ou de l'ADNc d'un échantillon à tester en tant que modèle, exécuter une amplification par PCR en une étape au moyen de la combinaison d'amorces selon l'une quelconque des revendications 1 à 6 ou du kit selon la revendication 7 pour obtenir un produit amplifié, le produit amplifié étant la banque d'amplicons du gène cible.

10. Procédé selon la revendication 9, dans lequel l'échantillon à tester est un échantillon de tissu in vitro, un échantillon congelé, un échantillon de ponction, un échantillon FFPE, du sang, de l'urine, du liquide cérébrospinal ou du liquide pleural.

11. Procédé de détection d'une mutation d'un gène cible d'un échantillon à tester, comprenant les étapes suivantes :

1) préparation d'une banque d'amplicons du gène cible d'après le procédé selon la revendication 9 ;
2) mélange homogène des banques d'amplicons des gènes cibles de tous les échantillons, puis dilution pour obtenir une banque d'ADN pour séquençage ;
3) séquençage de la banque d'ADN pour séquençage afin d'obtenir un résultat de séquençage, et analyse de la variation du gène cible de l'échantillon à tester selon le résultat de séquençage.

12. Procédé selon la revendication 11, dans lequel l'échantillon à tester est un échantillon de tissu in vitro, un échantillon congelé, un échantillon de ponction, un échantillon FFPE, du sang, de l'urine, du liquide cérébrospinal ou du liquide pleural.

13. Procédé de détection d'une fréquence de mutation dans une région cible d'un échantillon à tester, comprenant les étapes suivantes :

1) préparation d'une banque d'amplicons du gène cible au moyen du procédé selon la revendication 9 ;
2) mélange homogène des banques d'amplicons des gènes cibles de tous les échantillons, puis dilution pour obtenir une banque d'ADN pour séquençage ;
3) séquençage de la banque d'ADN pour séquençage afin d'obtenir un résultat de séquençage, et calcul de la fréquence de mutation du gène cible de l'échantillon à tester selon le résultat de séquençage ;

dans lequel la fréquence de variation = nombre de clusters mutationnels / nombre total de clusters efficaces $\times$ 100 %.

14. Procédé selon la revendication 13, dans lequel l'échantillon à tester est un échantillon de tissu in vitro, un échantillon congelé, un échantillon de ponction, un échantillon FFPE, du sang, de l'urine, du liquide cérébrospinal ou du liquide pleural.

FIG. 1

Forward outer primer F1  5'- [adapter sequence 1] [Barcode sequence] [universal sequence 1] -3'

Forward inner primer F2  5'- [universal sequence 1] [UMI molecular tag] [specific primer sequence] -3'

Reverse primer R  5'- [adapter sequence 2] [specific primer sequence] -3'

FIG. 2

FIG. 3

FIG. 4

FIG. 5

Triple-functional primer

Quadruple-functional primer

R

R1

R2

FIG. 6

Triple-functional component primer

Quadruple-functional component primer

FIG. 7

FIG. 8

FIG. 9

FIG. 10

FIG. 11